(19) Europäisches Patentamt
European Patent Office
Office européen des brevets

(11) EP 4 628 106 A1

(12) EUROPEAN PATENT APPLICATION
published in accordance with Art. 153(4) EPC

(43) Date of publication:
**08.10.2025 Bulletin 2025/41**

(21) Application number: **23896934.9**

(22) Date of filing: **01.12.2023**

(51) International Patent Classification (IPC):
*A61K 51/08 (2006.01)* *A61K 51/04 (2006.01)*
*C07D 207/46 (2006.01)* *A61K 31/16 (2006.01)*
*A61K 31/195 (2006.01)* *A61K 31/197 (2006.01)*
*A61K 31/165 (2006.01)* *C07K 5/078 (2006.01)*

(52) Cooperative Patent Classification (CPC):
**A61K 31/16; A61K 31/165; A61K 31/195; A61K 31/197; A61K 51/04; A61K 51/08; A61P 35/00; C07D 207/46; C07K 5/06139;**
A61K 51/00

(86) International application number:
**PCT/CN2023/135845**

(87) International publication number:
**WO 2024/114792 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.12.2022 CN 202211543739**

(71) Applicant: **Hexin (Suzhou) Pharmaceutical Technology Co., Ltd.**
**Suzhou, Jiangsu 215400 (CN)**

(72) Inventors:
- **SHAN, Changyu**
**Suzhou, Jiangsu 215400 (CN)**
- **ZENG, Dexing**
**Suzhou, Jiangsu 215400 (CN)**
- **CHEN, Yinfei**
**Suzhou, Jiangsu 215400 (CN)**
- **MAO, Liang**
**Suzhou, Jiangsu 215400 (CN)**

(74) Representative: **Schulz Junghans Patentanwälte PartGmbB**
**Großbeerenstraße 71**
**10963 Berlin (DE)**

Remarks:
The complete document including Reference Table(s) and the Sequence Listing(s) can be downloaded from the EPO website

# (54) HETERODIMER AND RADIOACTIVE MEDICAL USE THEREOF

(57) Provided is a compound comprising a structure as shown in the figure accompanying the abstract, and use thereof in radionuclide labeling or the preparation of a radionuclide labeling reagent. Also provided is a radionuclide preparation comprising the compound comprising the structure as shown in the figure accompanying the abstract.

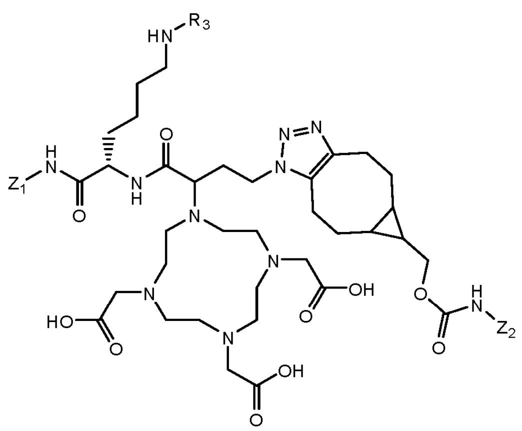

FIG. 10

EP 4 628 106 A1

## Description

### TECHNICAL FIELD

The present disclosure relates to the field of molecular imaging, and more particularly relates to a heterodimer and radioactive medical use thereof.

### BACKGROUND

#### Dual-target strategy

Currently, since personalized and precision medicine has gradually become a frontier in cancer treatment, the demand for new tumor-targeted imaging-therapy integration has emerged accordingly. At present, great progress has been made in tumor-targeted imaging research, highly specific peptides, antibodies, and nanoparticles are used as targeting molecules. By conjugating fluorescent, radionuclide, and other signaling molecules, non-invasive diagnosis of disease state in clinical cancer patients can be achieved, followed by targeted treatment. Although a series of single-receptor-targeting peptides exhibit good tumor-targeting properties in vivo, the single-receptor-targeting peptides have disadvantages such as low binding affinity, short tumor retention time, and insufficient tumor uptake. In some cases, the disadvantages will lead to suboptimal imaging contrast and specificity in tumor tissues, as well as a short probe retention time in tumor tissues, which are not conducive to tumor diagnosis and treatment.

Dual-targeting molecular probes fill the gap. Since many tumor cells overexpress multiple tumor-specific receptors on their surfaces, probes capable of recognizing multiple targets exhibit higher affinity and targeting efficiency than single-receptor probes. Dual-targeting molecular probes may simultaneously target two receptors/proteins expressed on cancer cell surfaces, a tumor microenvironment, or immune cells, and their specific interactions with two distinct targets enhanced their affinity. Due to the enhanced affinity and improved pharmacokinetic properties, the dual-targeting molecular probes exhibit superior specific uptake in tissues compared to the corresponding monomeric imaging agents. Therefore, low-affinity single-target ligands can be converted into high-affinity dual/multi-target ligands by leveraging multivalent interaction strategies to maximize binding capacity, which is a promising strategy for developing new highly-specific molecular probes for tumor diagnosis and treatment.

#### Integrin $\alpha_v\beta_3$ and CD13 receptors in tumor angiogenesis

Angiogenesis, a process of formation of new blood vessels from the existing ones, is widely recognized as a crucial pathway for rapidly growing tumor tissues to obtain nutrients and oxygen, and is also significantly associated with tumor cell invasion and metastasis. Since tumor growth and metastasis are highly dependent on angiogenesis, this phenomenon is considered to be a major target for tumor diagnosis and treatment. The process of tumor angiogenesis is stimulated by various growth factors, including integrin $\alpha_v\beta_3$ receptors and CD13 receptors.

Integrin $\alpha_v\beta_3$, a receptor for extracellular matrix proteins with an arginine-glycine-aspartic (RGD) tripeptide sequence, plays a significant role in tumor angiogenesis. These include vitronectin, fibronectin, fibrinogen, lamin, collagen, Von Willibrand's factor, osteoponin, and adenovirus particles. Integrin $\alpha_v\beta_3$ is expressed at low levels on epithelial cells and mature endothelial cells, but it is overexpressed on the activated endothelial cells of tumor neovasculature and some tumor cells. Studies have shown that integrin $\alpha_v\beta_3$ is highly correlated with tumor growth, invasion, and metastasis, making it a key molecular target for both early detection and treatment of rapidly growing solid tumors.

CD13 is a membrane glycoprotein that functions as an extracellular aminopeptidase, with a specific ligand containing the NGR tripeptide sequence. High expression of CD13 has been observed in various tumors, including pancreatic cancer, breast cancer, ovarian cancer, and melanoma. CD13 is also involved in tumor angiogenesis, as CD13 is present in tumor endothelial cells but absent in normal tissue blood vessels.

#### Radiopharmaceutical therapy of tumors

Targeted radiotherapy is an important tumor treatment method, especially for metastatic and highly metastasized cancers that are ineffective with surgery or traditional radiotherapy. Targeted radiotherapy uses carriers or interventional techniques to specifically concentrate radionuclides in diseased tissues or cells. The radiation particles emitted by radionuclides move through biological tissues, accompanied by energy transfer and ionization, and the energy generated therefrom can directly break chemical bonds of biomacromolecules, such as nucleic acids and proteins, resulting in changes in molecular structure and function, particularly DNA strand breakage and synthesis disorder could cause cell cycle arrest or apoptosis in damaged cells, thereby exerting a therapeutic effect. Targeted radiotherapy usually uses a targeting agent/carrier (for example, antibody, peptide or small molecule) labeled with therapeutic radioactive isotopes (for

example, $^{177}Lu$, $^{90}Y$, $^{131}I$, $^{89}Sr$, $^{32}P$ and the like that emit β rays, and $^{223}Ra$, $^{212}Bi$, $^{225}Ac$ and the like that emit α rays), to deliver cytotoxic doses of radioactive rays to target cancer cells. In order to achieve therapeutic effect, the commonly used therapeutic radionuclides usually have relatively long half-lives to provide sustained internal radiation. For example, $^{177}Lu$ has a half-life of 6.7 days, $^{131}I$ has a half-life of 8.1 days, and $^{32}P$ has a half-life of 14.3 days. In order to maximize the therapeutic effect of radionuclides, the biological half-life of the targeting agent/carrier should be designed to match the radioactive half-life of the paired radionuclides as much as possible. However, the polypeptide-based targeting carriers currently have the defects of low tumor uptake and rapid in vivo clearance rate. Therefore, radiotherapy using polypeptides as the targeting carrier usually leads to insufficient radiation doses at tumor tissues, limiting their clinical applications in tumor treatment.

**Albumin-based carrier strategy**

Albumin has been extensively explored as drug delivery carriers owing to its advantages, including high biocompatibility, strong non-antigenicity, good biodegradability, and easy surface modification. To date, several albumin-based nanosized drug delivery systems have successfully transitioned into clinical applications, which significantly improved pharmacokinetics and tumor accumulation of therapeutics, thereby improving therapeutic efficiency and minimizing side effects of drugs.

In the field of tumor radiotherapy, molecular design can incorporate albumin-binding fragments into carrier molecules, so that the targeting molecules labeled with radionuclides can bind to albumin in vivo, blood circulation time and half-life of the targeting molecules are greatly prolonged, and pharmacokinetic properties are also improved. In addition, a large molecular size of the albumin and EPR effect of tumor tissues can be used to enhance the targeting efficiency of targeting probe and radioactive isotopes. Since a tumor tissue environment exhibits high metabolic level and a high uptake of nutrients such as albumin, the tumor uptake of targeting probes and radioactive isotopes can be accordingly increased.

However, the use of albumin-based loading technology may also lead to an increase in uptake of radioactive targeting peptides by healthy organs, resulting in corresponding side effects.

**SUMMARY**

In some embodiments, provided is a compound, comprising or consisting of a structure shown in Formula (A).

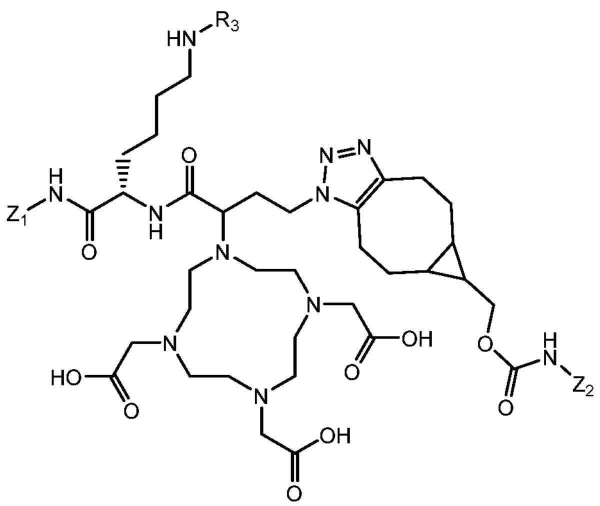

Formula (A),

wherein, $Z_1$ is $-L_1R_1$ or -H, $Z_2$ is $-L_2R_2$ or -H, in the provisos that $Z_1$ is $-L_1R_1$ and/or $Z_2$ is $-L_2R_2$; $L_1$ is $-(PEG)_m-$, and *m* is an integer selected from 4 to 30; $L_2$ is $-(PEG)_n-$, and *n* is an integer selected from 4 to 30; $R_1$ comprises a peptide sequence sequentially connected by arginine, glycine or sarcosine, and aspartic acid; $R_2$ comprises a peptide sequence sequentially connected by asparagine, glycine or sarcosine, cysteine, and arginine; $R_3$ is a group of Formula (I) or a group of Formula (II); $R_4$ is a group of Formula (III), a group of Formula (IV), or a long-chain fatty acid; $Q_1$, $Q_2$, $Q_3$, $Q_4$, and $Q_5$ are each independently selected from a group consisting of -H, -F, -Cl, -Br, -I, $C_1$-$C_6$ linear or branched alkyl, $C_1$-$C_6$ linear or branched fluoroalkyl, and $C_1$-$C_6$ linear or branched fluoroalkoxy; and x is an integer selected from a group consisting of 1, 2, 3, 4, 5, 6, 7, and 8.

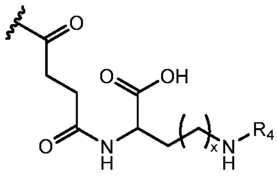 Formula (I) 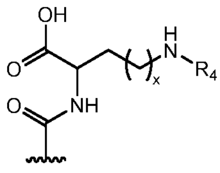 Formula (II)

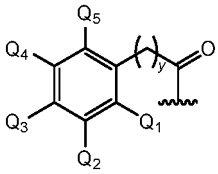 Formula (III) 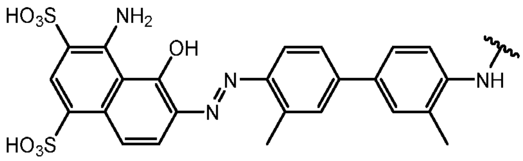 Formula (IV)

In some embodiments, provided is a radionuclide preparation, comprising or consisting of the compound and a radionuclide chelated thereby.

In some embodiments, the present disclosure provides preparation methods of the compound or the radionuclide in the present disclosure, comprising: obtaining a first compound comprising a structure of Formula (A1); obtaining a second compound comprising a structure of Formula (A2); forming an amide bond through an -$N^*H_2$ group of the second compound and a -C*OOH group of a compound of Formula (I') or Formula (II') or an -$N^*H_2$ group protected compound thereof, to obtain a third compound comprising a group of Formula (I") or a group of Formula (II"); and reacting between the third compound and the first compound, to obtain a fourth compound; and forming an amide bond through an -$N^*H_2$ group in the group of Formula (I") or Formula (II") in the fourth compound and a -C*OOH group of a compound of Formula (III').

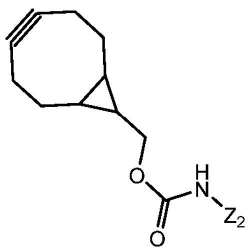 Formula (A1) 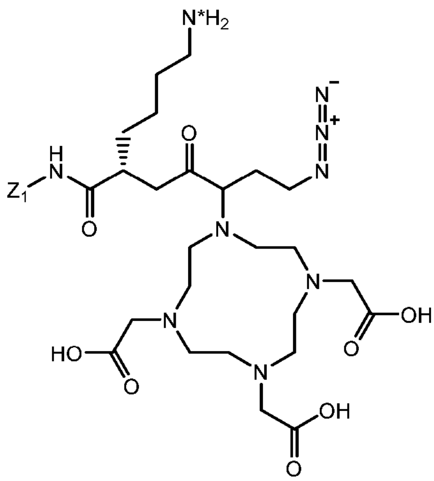 Formula (A2)

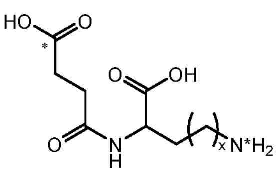 Formula (I') 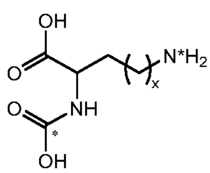 Formula (II') 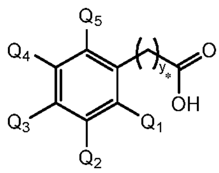 Formula (III')

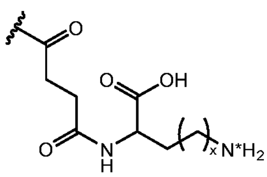 Formula (I'') 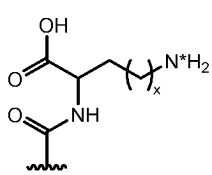 Formula (II'')

In some embodiments, provided is use of the compound or the radionuclide preparation of the present disclosure in the preparation of a medicament for detecting cancer, diagnosing cancer, monitoring cancer progression, monitoring cancer treatment, or treating cancer.

In some embodiments, provided is use of the compound of the present disclosure in radionuclide labeling. In some embodiments, provided is use of the compound of the present disclosure is preparing a radionuclide-labeled

targeting molecule. In some embodiments, provided is use of the compound of the present disclosure in the preparation of a radionuclide labeling reagent. In some embodiments, provided is use of the compound of the present disclosure in preparing a drug carrier. In some embodiments, provided is use of the compound of the present disclosure as a drug carrier. In some embodiments, provided is use of the compound or the radionuclide preparation of the present disclosure in the preparation of a medicament for detecting cancer, diagnosing cancer, monitoring cancer progression, monitoring cancer treatment, or treating cancer. In some embodiments, provided is use of the compound or the radionuclide preparation of the present disclosure in detecting cancer, diagnosing cancer, monitoring cancer progression, monitoring cancer treatment, or treating cancer.

In some embodiments, provided is use of the compound of the present disclosure for use in radionuclide labeling. In some embodiments, provided is a radionuclide-labeled targeting molecule, comprising or consisting of the compound of the present disclosure. In some embodiments, provided is a radionuclide labeling reagent, comprising or consisting of the compound of the present disclosure. In some embodiments, provided is a drug carrier, comprising or consisting of the compound of the present disclosure. In some embodiments, provided is a formulation for use in detecting cancer, diagnosing cancer, monitoring cancer progression, monitoring cancer treatment, or treating cancer, comprising or consisting of the compound or the radionuclide preparation of the present disclosure. In some embodiments, provided is a medicament for detecting cancer, diagnosing cancer, monitoring cancer progression, monitoring cancer treatment, or treating cancer, comprising the compound or the radionuclide preparation of the present disclosure.

In some embodiments, provided is a method of radionuclide labeling, comprising administering the compound chelated with the radionuclide, or the radionuclide preparation. In some embodiments, provided is a radionuclide labeling method, comprising bringing the compound chelated with the radionuclide, or the radionuclide preparation into contact with an object to be labeled by the radionuclide. In some embodiments, provided is a method for detecting cancer, diagnosing cancer, monitoring cancer progression, monitoring cancer treatment, or treating cancer, comprising: administering the compound chelated with the radionuclide or the radionuclide preparation to a subject for detecting cancer, diagnosing cancer, monitoring cancer progression, or monitoring cancer treatment; detecting the radionuclide and determining level and location of the radionuclide in the subject; comparing level and location of the radionuclide with levels and locations of the radionuclide in a same location as other aspects of an unaffected subject or an unaffected part of the subject, where compared with the level and location of the radionuclide in a sample from the radionuclide from the unaffected subject or the unaffected part of the subject, a higher level or different location of the radionuclide in the subject indicates that the presence of a cancer in the subject, thereby detecting cancer, diagnosing cancer, monitoring cancer progression, or monitoring cancer treatment. In some embodiments, provided is a method of treating cancer in a patient, comprising administering the compound chelated with the radionuclide, or the radionuclide preparation. In some embodiments, provided is a method of treating cancer in a patient, comprising contacting the compound chelated with the radionuclide, or the radionuclide preparation with the patient.

## BRIEF DESCRIPTION OF THE DRAWINGS

FIG. 1 is an electrospray ionization mass spectrometry (ESI-MS) diagram of a precursor NGR-$PEG_4$-BCN according to some embodiments of the present disclosure.

FIG. 2 is an electrospray ionization mass spectrometry diagram of a precursor $RG_D$-$PEG_4$-Lys-DOTA-$N_3$ according to some embodiments of the present disclosure.

FIG. 3 is an electrospray ionization mass spectrometry diagram of a precursor $RG_D$-$PEG_4$-Lys(-R-H)-DOTA-$N_3$ according to some embodiments of the present disclosure.

FIG. 4 is an electrospray ionization mass spectrometry diagram of a precursor $RG_D$-$PEG_4$-Lys(-R-H)-DOTA-click-$PEG_4$-NGR according to some embodiments of the present disclosure.

FIG. 5 is an electrospray ionization mass spectrometry diagram of a compound $RG_D$-$PEG_4$-Lys (-R-$R_1$)-DOTA-click-$PEG_4$-NGR according to some embodiments of the present disclosure.

FIG. 6 shows uptake of complexes $^{68}$Ga-L00, $^{68}$Ga-L11, $^{68}$Ga-L12, $^{68}$Ga-L13 and $^{68}$Ga-L21 in tumors, muscles, and kidneys at various time points after intravenous injection according to some embodiments of the present disclosure, where **A** shows uptake of tumors, **B** shows uptake of muscles, **C** shows uptake of kidneys, and **D** shows comparison of uptake of complexes $^{68}$Ga-L11, $^{68}$Ga-L12, $^{68}$Ga-L13 and $^{68}$Ga-L21 after 5 hours. A vertical axis denotes a standard uptake value (SUV).

FIG. 7 shows uptake of complexes $^{68}$Ga-L00, $^{68}$Ga-L11, $^{68}$Ga-L12, $^{68}$Ga-L13 and $^{68}$Ga-L21 in tumors, muscles, and kidneys at various time points after intravenous injection according to some embodiments of the present disclosure, where **A** shows distribution of drug in vivo 5 hours after injection in a representative animal selected from each conjugate group; **B** shows a tumor/muscle ratio of each conjugate group at each time point (tumor uptake divided by muscle uptake, expressed as a percentage); and **C** shows a tumor/kidney ratio of each conjugate group at each time

point (tumor uptake divided by kidney uptake, expressed as a percentage).

FIG. 8 is static SPECT-CT images of mice 4, 24, 48, 72 and 96 hours after intravenous injection of complex $^{177}$Lu-L11.

FIG. 9 shows distribution of radioactivity in various tissues of mice 24 hours after intravenous injection of complexes $^{177}$Lu-L11, $^{177}$Lu-L12, $^{177}$Lu-$_D$-L11 and $^{177}$Lu-L11-1.

FIG. 10 is Formula (A).

## DETAILED DESCRIPTIONS OF THE EMBODIMENTS

As described herein, singular terms refer to one or more than one. For example, "element" or "an element" refers to one element or more than one element.

As described herein, the term "about" refers to approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. In some cases, the term "about" refers to a numerical variation of plus or minus 20% of the numerical values set forth. For example, "about 50%" refers to within a range of 45%-55%. Numerical ranges recited herein by endpoints include all numbers and fractions subsumed within that range (for example, 1 to 5 includes 1, 1.5, 2, 2.75, 3, 3.90, 4, and 5). It should also be understood that all numbers and fractions thereof are presumed to be modified by the term "about."

As used therein, the terms "comprising" or "including" are intended to indicate that a combination (such as device, composition, and method) includes the listed elements (such as units of a device, constituents of a composition, or substantial steps of a method), but does not exclude other elements. When defining a composition or method, the phrase "consisting essentially of" refers to excluding other elements that are of significant importance to the combination for its intended purpose. Therefore, a combination consisting essentially of the elements defined herein does not exclude other elements that do not substantially affect the fundamental and novel features of the present disclosure. The term "consisting of" refers to a combination that excludes other elements (constituent components or substantial method steps). Embodiments defined by each of these transitional phrases fall within the scope of the present disclosure.

The term "amino acid" may be used interchangeably with "amino acid residue," and may refer to a free amino acid and/or to an amino acid residue of a peptide. It will be apparent from the context in which the term is used whether it refers to a free amino acid or a residue of a peptide. An "amino acid" As described herein is meant to include both natural and synthetic amino acids, and both $_D$- and $_L$- amino acids. The term "standard amino acid" refers to any of the twenty standard amino acids (including glycine) commonly found in naturally occurring peptides. As described herein, the terms "$_D$-form" and "$_L$-form" amino acids are not intended to exclude achiral amino acids such as glycine, unless otherwise specified. The term "non-standard amino acid residue" refers to any amino acid, other than the standard amino acids, regardless of whether it is prepared synthetically or derived from a natural source. As described herein, "synthetic amino acid" further encompasses chemically modified amino acids, including but not limited to salts, amino acid derivatives (such as amides), and substitutions. Amino acids contained within the peptides of the present disclosure, and particularly at the at the C- or N-terminal, can be modified by methylation, amidation, acetylation or substitution with other chemical groups which can change a peptide's circulating half-life without adversely affecting activity of the peptide. In addition, disulfide bonds may be present or absent in the peptides of the present disclosure.

As described herein, the term "pharmaceutical composition" refers to a composition containing at least one active ingredient, where the composition is acceptable for achieving a specific, effective result in mammals (for example, including but not limited to human being). Based on the needs of the skilled artisan, those of ordinary skill in the art will recognize and understand the technology suitable for determining whether an active ingredient exhibits the desired efficacy.

As described herein, the term "pharmaceutically acceptable carrier" refers to a chemical composition to which a suitable compound or derivative can be combined and which, after combination, can be used to administer a suitable compound to a subject.

As described herein, the term "physiologically acceptable" ester or salt refers to an ester or salt form of the active ingredient that is compatible with any other ingredients of a pharmaceutical composition, which is not deleterious to the subject receiving the composition.

As described herein, the term "pharmaceutically acceptable" refers to physiologically tolerable, for either human or veterinary use.

As described herein, the term "pharmaceutical composition" includes formulations intended for both human and veterinary use.

As described herein, the term "a plurality of" refers to at least two.

As described herein, the term "polynucleotide" refers to a single strand or parallel and antiparallel strands of nucleic acid. Therefore, a polynucleotide may be a single-stranded or double-stranded nucleic acid.

As described herein, the term "polypeptide" refers to a polymer composed of amino acid residues, related

naturally occurring structural variants, and synthetic non-naturally occurring analogs thereof linked via peptide bonds, related naturally occurring structural variants, and synthetic non-naturally occurring analogs thereof.

As described herein, the term "synthetic peptides or polypeptides" refers to non-naturally occurring peptides and polypeptides. For example, a synthetic peptide or polypeptide can be synthesized by an automatic peptide synthesizer.

As described herein, the term "and/or" refers to and encompasses any and all possible combinations of one or more relevant listed items. When being used in a list of two or more items, the term "and/or" refers to that any of the listed items may be used alone, or that any combination of two or more listed items may be used. For example, when a composition, combination, construction, and the like, is described as including (or containing) components A, B, C, and/or D, the composition may contain A alone, contain B alone, contain C alone, contain D alone; or contain a combination of A and B, contain a combination of A and C, contain a combination of A and D, contain a combination of B and C, contain a combination of B and D, contain a combination of C and D; as well as contain a combination of A, B and C, contain a combination of A, B and D, contain a combination of A, C and D, and contain a combination of B, C and D, or contain a combination of A, B, C and D.

As described herein, the compounds or ions of the present disclosure include a plurality of variable groups. Those skilled in the art will recognize that the combinations of groups envisioned in the present disclosure are combinations of compounds or ions that are chemically permissible.

As described herein, the stereochemistry of a chiral center can be defined according to the conventions of those skilled in the art, that is, using "" (a wedged bond) to indicate a group oriented outward from the plane of the paper (towards the reader), and using "" (a hashed bond) to indicate a group oriented inward from the plane of the paper (away from the reader). If such a representation is used, it can be understood as indicating a specific stereoisomer of the group shown in each chemical structure herein. Any bond not specifically represented by a wedge bond or a hashed bond herein should be deemed as not specifically indicating whether the bond is oriented outward from the plane of the paper, inward from the plane of the paper, or in the plane of the paper, but does not preclude its being oriented outward or inward from the plane of the paper where chemically permissible.

As described herein, the term "isomer" refers to compounds that have a same molecular formula but differ in the nature of order of bonding of their atoms or the arrangement of their atoms in space. Specifically, the term "stereoisomer" refers to isomers with different arrangements of atoms in space; the term "enantiomer" refers to stereoisomers with one or more asymmetric centers that are non-superimposable mirror images of one another; and the term "diastereomer" refers to stereoisomers that are not enantiomers but have opposite configurations at one or more asymmetric centers. When a compound has an asymmetric center, for example, when a carbon atom is bonded to four different groups, a pair of enantiomers can exist. An enantiomer can be characterized by an absolute configuration of one or more asymmetric centers, and designated as R-configuration or S-configuration, or designated as dextrorotatory or levorotatory by a manner in which the molecule rotates the plane of polarized light. Chiral compounds can exist as an individual enantiomer or as a mixture thereof, such as a racemic mixture. The compounds of the present disclosure may contain asymmetric or chiral centers, and therefore exist in different stereoisomeric forms. It is intended that all stereoisomeric forms of the compounds of the present disclosure, including but not limited to, diastereomers, enantiomers and atropisomers, as well as mixtures thereof such as racemic mixtures, form part of the present disclosure.

As described herein, especially with respect to the atoms shown in the structural formulas herein should be deemed to include all isotopes of the atoms. For example, irrespective of being shown in text or in structural formulas or omitted, hydrogen atoms (H) herein should be deemed to include all isotopes of hydrogen, such as, including but not limited to, protium, deuterium, tritium, and the like. Similarly, irrespective of being shown in text or in structural formulas, carbon atoms (C) should be deemed to include all isotopes of carbon, such as, including but not limited to, $^{12}C$, $^{13}C$, $^{14}C$, and the like.

As described herein, especially in the structural formulae herein, when the symbol "*" is used to mark an atom, it is only and solely used to mark a specific atom in the structural formula for the purpose of clarification in the present disclosure, and does not imply that the atom marked by it has different properties that are not described elsewhere in the present disclosure.

The expression "$(PEG)_n$" as used herein refers to polyethylene glycol composed of n ethylene glycol monomers. Similarly, the expression "$(PEG)_m$," as used herein follows the same principle. For example, the expression "$(PEG)_4$" as used herein refers to polyethylene glycol composed of 4 ethylene glycol monomers.

In some embodiments, $Z_1$ is $-L_1R_1$. In some embodiments, $Z_1$ is H. In some embodiments, $Z_2$ is $-L_2R_2$. In some embodiments, $Z_2$ is -H. In some embodiments, $Z_1$ is $-L_1R_1$ and/or $Z_2$ is $-L_2R_2$. In some embodiments, $Z_1$ is $-L_1R_1$ and $Z_2$ is $-L_2R_2$. In some embodiments, $Z_1$ is $-L_1R_1$ and $Z_2$ is -H. In some embodiments, $Z_1$ is -H and $Z_2$ is $-L_2R_2$.

In some embodiments, *m* is an integer selected from 4 to 30. In some embodiments, *m* is an integer selected from 4 to 25. In some embodiments, *m* is an integer selected from 4 to 20. In some embodiments, *m* is an integer selected from 4 to 15. In some embodiments, *m* is an integer selected from 4 to 10. In some embodiments, *m* is selected from a group consisting of 4, 5, 6, 7, and 8. In some embodiments, *m* is selected from a group consisting of 4, 5, and 6. In some embodiments, *m* is 4 or 5. In some embodiments, *m* is 4. In some embodiments, *m* is 5. In some embodiments, *m* is 6. In

some embodiments, *m* is 7. In some embodiments, *m* is 8. In some embodiments, *m* is 9. In some embodiments, *m* is 10. In some embodiments, *m* is 11. In some embodiments, *m* is 12. In some embodiments, *m* is 13. In some embodiments, *m* is 14. In some embodiments, *m* is 15. In some embodiments, *m* is 16. In some embodiments, *m* is 17. In some embodiments, *m* is 18. In some embodiments, *m* is 19. In some embodiments, *m* is 20. In some embodiments, *m* is 21. In some embodiments, *m* is 22. In some embodiments, *m* is 23. In some embodiments, *m* is 24. In some embodiments, *m* is 25. In some embodiments, *m* is 26. In some embodiments, *m* is 27. In some embodiments, *m* is 28. In some embodiments, *m* is 29. In some embodiments, *m* is 30.

In some embodiments, *n* is an integer selected from 4 to 30. In some embodiments, *n* is an integer selected from 4 to 25. In some embodiments, *n* is an integer selected from 4 to 20. In some embodiments, *n* is an integer selected from 4 to 15. In some embodiments, *n* is an integer selected from 4 to 10. In some embodiments, *n* is selected from a group consisting of 4, 5, 6, 7, and 8. In some embodiments, *n* is selected from a group consisting of 4, 5, and 6. In some embodiments, *n* is 4 or 5. In some embodiments, *n* is 4. In some embodiments, *n* is 5. In some embodiments, *n* is 6. In some embodiments, *n* is 7. In some embodiments, *n* is 8. In some embodiments, *n* is 9. In some embodiments, *n is* 10. In some embodiments, *n is* 11. In some embodiments, *n is* 12. In some embodiments, *n* is 13. In some embodiments, *n* is 14. In some embodiments, *n* is 15. In some embodiments, *n* is 16. In some embodiments, *n* is 17. In some embodiments, *n* is 18. In some embodiments, *n* is 19. In some embodiments, *n* is 20. In some embodiments, *n* is 21. In some embodiments, *n* is 22. In some embodiments, *n* is 23. In some embodiments, *n* is 24. In some embodiments, *n* is 25. In some embodiments, *n* is 26. In some embodiments, *n* is 27. In some embodiments, *n* is 28. In some embodiments, *n* is 29. In some embodiments, *n* is 30.

In some embodiments, $R_1$ comprises a tripeptide sequence sequentially connected by arginine, glycine or sarcosine, and aspartic acid. In some embodiments, $R_1$ comprises a tripeptide sequence sequentially connected by L-arginine, glycine or sarcosine, and L-aspartic acid. In some embodiments, $R_1$ comprises a tripeptide sequence sequentially connected by L-arginine, glycine, and L-aspartic acid. In some embodiments, $R_1$ comprises a tripeptide sequence sequentially connected by L-arginine, sarcosine, and L-aspartic acid. In some embodiments, $R_1$ is a cyclic peptide. In some embodiments, $R_1$ is a cyclic peptide consisting of 4 to 7 amino acids. In some embodiments, $R_1$ is a cyclic peptide consisting of 5 amino acids. In some embodiments, $R_1$ further comprises lysine. In some embodiments, $R_1$ is a cyclic peptide comprising lysine. In some embodiments, $R_1$ is a cyclic peptide consisting of 4 to 7 amino acids comprising lysine. In some embodiments, $R_1$ is a cyclic peptide consisting of 5 amino acids comprising lysine. In some embodiments, $R_1$ is a cyclic peptide shown in any one of SEQ ID NO: 1-2. In some embodiments, $R_1$ is a cyclic peptide shown in SEQ ID NO: 1. In some embodiments, $R_1$ is a cyclic peptide shown in SEQ ID NO: 2.

In some embodiments, $R_2$ comprises a tripeptide sequence sequentially connected by asparagine, glycine or sarcosine, and arginine. In some embodiments, $R_2$ comprises a tripeptide sequence sequentially connected by L-asparagine, glycine or sarcosine, and L-arginine. In some embodiments, $R_2$ comprises a tripeptide sequence sequentially connected by L-asparagine, glycine, and L-arginine. In some embodiments, $R_2$ comprises a tripeptide sequence sequentially connected by asparagine, sarcosine, and L-arginine. In some embodiments, $R_2$ is a cyclic peptide. In some embodiments, $R_2$ is a cyclic peptide consisting of 4-7 amino acids. In some embodiments, $R_2$ is a cyclic peptide consisting of 6 amino acids. In some embodiments, $R_2$ further comprises lysine. In some embodiments, $R_2$ is a cyclic peptide comprising lysine. In some embodiments, $R_2$ is a cyclic peptide consisting of 4-7 amino acids comprising lysine. In some embodiments, $R_2$ is a cyclic peptide consisting of 6 amino acids comprising lysine. In some embodiments, $R_2$ is a cyclic peptide shown in any one of SEQ ID NO: 3-6. In some embodiments, $R_2$ is a cyclic peptide shown in SEQ ID NO: 3. In some embodiments, $R_2$ is a cyclic peptide shown in SEQ ID NO: 4. In some embodiments, $R_2$ is a cyclic peptide shown in SEQ ID NO: 5. In some embodiments, $R_2$ is a cyclic peptide shown in SEQ ID NO: 6.

In some embodiments, $R_3$ is a group of Formula (I). In some embodiments, $R_3$ is a group of Formula (II).

In some embodiments, the group of Formula (I) is a group of Formula (L-I) or a group of Formula (D-I). In some embodiments, the group of Formula (II) is a group of Formula (L-II) or a group of Formula (D-II). In some embodiments, the group of R is a group of Formula (L-I). In some embodiments, the group of R is a group of Formula (D-I). In some embodiments, the group of R is a group of Formula (L-II). In some embodiments, the group of R is a group of Formula (D-II). In some embodiments, the group of R is selected from a group consisting of a group of Formula (L-I), a group of Formula (D-I), a group of Formula (L-II), and a group of Formula (D-II).

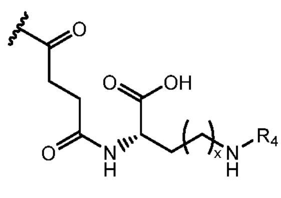
Formula (L-I)

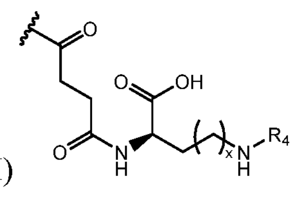
Formula (D-I)

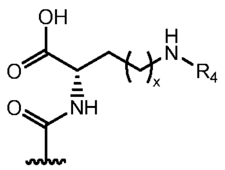
Formula (L-II)
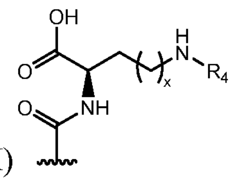
Formula (D-II)

In some embodiments, the compound of Formula (I') is a compound of Formula (L-I') or a group of Formula (D-I'). In some embodiments, the compound of Formula (II') is a compound of Formula (L-II') or a group of Formula (D-II'). In some embodiments, the compound of Formula (I') is a compound of Formula (L-I'). In some embodiments, the compound of Formula (I') is a compound of Formula (D-I'). In some embodiments, the compound of Formula (II') is a compound of Formula (L-II'). In some embodiments, the compound of Formula (II') is a compound of Formula (D-II). In some embodiments, the group of Formula (I") is a group of Formula (L-I") or a group of Formula (D-II"). In some embodiments, the group of Formula (II") is a group of Formula (L-II") or a group of Formula (D-II). In some embodiments, the group of Formula (II") is a group of Formula (L-I"). In some embodiments, the group of Formula (II") is a group of Formula (D-I"). In some embodiments, the group of Formula (II") is a group of Formula (L-I"). In some embodiments, the group of Formula (II") is a group of Formula (D-II).

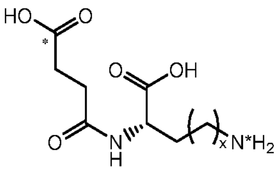
Formula (L-I')
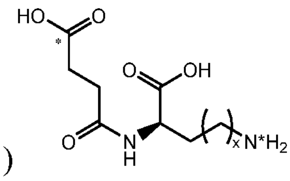
Formula (D-I')

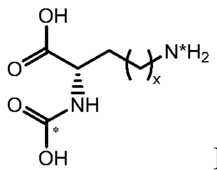
Formula (L-II')
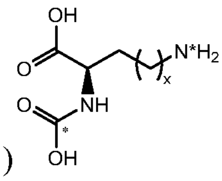
Formula (D-II')

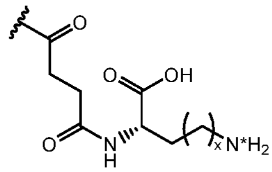
Formula (L-I")
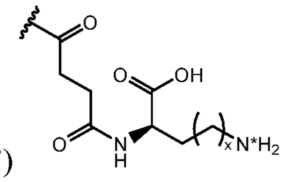
Formula (D-I")

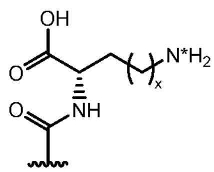
Formula (L-II")
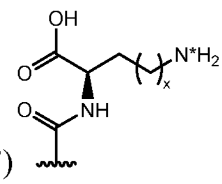
Formula (D-II")

In some embodiments, $R_4$ is a group of Formula (III). In some embodiments, $R_4$ is a group of Formula (IV). In some embodiments, $R_4$ is a long-chain fatty acid.

In some embodiments, $Q_1$, $Q_2$, $Q_3$, $Q_4$, and $Q_5$ are each independently selected from a group consisting of -H, -F, -Cl, -Br, -I, $-CH_2F$, $-CHF_2$, and $-CF_3$. In some embodiments, $Q_3$ is selected from a group consisting of -H, -F, -Cl, -Br, -I, $-CH_2F$, $-CHF_2$, and $-CF_3$. In some embodiments, one of $Q_1$, $Q_2$, $Q_3$, $Q_4$ and $Q_5$ is -F. In some embodiments, two of $Q_1$, $Q_2$, $Q_3$, $Q_4$, and $Q_5$ are -F. In some embodiments, $Q_1$ is -H. In some embodiments, $Q_2$ is -H. In some embodiments, $Q_3$ is -F. In some embodiments, $Q_3$ is -Cl. In some embodiments, $Q_3$ is -Br. In some embodiments, $Q_3$ is -I. In some embodiments, $Q_3$ is $-CH_2F$. In some embodiments, $Q_3$ is $-CHF_2$. In some embodiments, $Q_3$ is $-CF_3$. In some embodiments, $Q_4$ is -F. In some embodiments, $Q_5$ is -F. In some embodiments, $Q_4$ is -H and $Q_5$ is -H. In some embodiments, $Q_4$ is -H and $Q_5$ is -F. In some embodiments, $Q_4$ is -H and $Q_5$ is -H. In some embodiments, $Q_4$ is -F and $Q_5$ is -F.

In some embodiments, x is selected from a group consisting of 1, 2, 3, 4, 5, 6, 7, and 8. In some embodiments, x is selected from a group consisting of 1, 2, 3, 4, 5, 6, and 7. In some embodiments, x is selected from a group consisting of 1, 2,

3, 4, 5, and 6. In some embodiments, x is selected from a group consisting of 1, 2, 3, 4, and 5. In some embodiments, x is selected from a group consisting of 1, 2, 3, and 4. In some embodiments, x is selected from a group consisting of 1, 2, and 3. In some embodiments, *x* is selected from a group consisting of 2, 3, and 4. In some embodiments, *x* is selected from a group consisting of 3, 4, and 5. In some embodiments, *x* is 2 or 3. In some embodiments, *x* is 3 or 4. In some embodiments, *x* is 1. In some embodiments, *x* is 2. In some embodiments, *x* is 3. In some embodiments, *x* is 4. In some embodiments, *x* is 5. In some embodiments, *x* is 6. In some embodiments, *x* is 7. In some embodiments, *x* is 8.

In some embodiments, *y* is selected from a group consisting of 1, 2, 3, 4, 5, and 6. In some embodiments, *y* is selected from a group consisting of 1, 2, 3, 4, and 5. In some embodiments, *y* is selected from a group consisting of 1, 2, 3, and 4. In some embodiments, *y* is selected from a group consisting of 1, 2, and 3. In some embodiments, *y* is selected from a group consisting of 2, 3, and 4. In some embodiments, *y* is selected from a group consisting of 3, 4, and 5. In some embodiments, *y* is selected from a group consisting of 4, 5, and 6. In some embodiments, *y* is 2 or 3. In some embodiments, *y* is 3 or 4. In some embodiments, *y* is 1. In some embodiments, *y* is 2. In some embodiments, *y* is 3. In some embodiments, *y* is 4. In some embodiments, *y* is 5. In some embodiments, *y* is 6.

In some embodiments, *y* is 3, $Q_1$ is -H, $Q_2$ is -H, $Q_3$ is $-CF_3$, $Q_4$ is -H or -F, and $Q_5$ is -H or - F. In some embodiments, *x* is 3, *y* is 3, $Q_1$ is -H, $Q_2$ is -H, $Q_3$ is $-CF_3$, $Q_4$ is -H or -F, and $Q_5$ is -H or -F. In some embodiments, $R_4$ is a group of Formula (IIIa), $Q_4$ is -H or -F, and $Q_5$ is -H or -F.

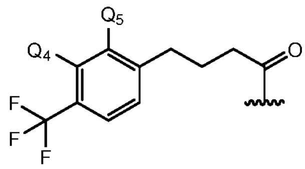

Formula (IIIa)

In some embodiments, $R_3$ is a group of Formula (I), *x* is 3, $R_4$ is a group of Formula (IIIa), $Q_4$ is H, and $Q_5$ is H. In some embodiments, $R_3$ is a group of Formula (I), *x* is 3, $R_4$ is a group of Formula (IIIa), $Q_4$ is F, and $Q_5$ is H. In some embodiments, $R_3$ is a group of Formula (I), *x* is 3, $R_4$ is a group of Formula (IIIa), $Q_4$ is H, and $Q_5$ is F. In some embodiments, $R_3$ is a group of Formula (I), *x* is 3, $R_4$ is a group of Formula (IIIa), $Q_4$ is F, and $Q_5$ is F. In some embodiments, $R_3$ is a group of Formula (II), *x* is 3, $R_4$ is a group of Formula (IIIa), $Q_4$ is H, and $Q_5$ is H. In some embodiments, $R_3$ is a group of Formula (II), *x* is 3, $R_4$ is a group of Formula (IIIa), $Q_4$ is F, and $Q_5$ is H. In some embodiments, $R_3$ is a group of Formula (II), *x* is 3, $R_4$ is a group of Formula (IIIa), $Q_4$ is H, and $Q_5$ is F. In some embodiments, $R_3$ is a group of Formula (II), *x* is 3, $R_4$ is a group of Formula (IIIa), $Q_4$ is F, and $Q_5$ is F.

In some embodiments, $Z_1$ is $-L_1R_1$ and $Z_2$ is $-L_2R_2$; and/or *m* is 4; and/or *n* is 4. In some embodiments, *x* is 3; and/or $R_4$ is a group of Formula (III), and *y* is 2 or 3. In some embodiments, $R_4$ is a group of Formula (III); and $Q_1$ is -H; $Q_2$ is -H; $Q_3$ is selected from a group consisting of - H, -F, -Cl, -Br, -I, $-CH_2F$, $-CHF_2$, and $-CF_3$; $Q_4$ is -H or -F; and/or $Q_5$ is -H or -F.

In some embodiments, the compound of the present disclosure chelates a radionuclide. In some embodiments, the radionuclide comprises or consists of at least one selected from a group consisting of $^{44}Sc$, $^{47}Sc$, $^{62}Cu$, $^{64}Cu$, $^{67}Cu$, $^{66}Ga$, $^{67}Ga$, $^{68}Ga$, $^{86}Y$, $^{90}Y$, $^{89}Zr$, $^{99m}Tc$, $^{110}In$, $^{111}In$, $^{113m}In$, $114^{m}In$, $^{177}Lu$, $^{188}Re$, $^{203}Pb$, $^{212}Pb$, $^{212}Bi$, $^{213}Bi$, $^{211}At$, $^{223}Ra$, and $^{225}Ac$. In some embodiments, the radionuclide comprises or consists of at least one selected from a group consisting of $^{44}Sc$, $^{47}Sc$, $^{64}Cu$, $^{67}Cu$, $^{67}Ga$, $^{68}Ga$, $^{90}Y$, $^{99m}Tc$, $^{111}In$, $^{177}Lu$, $^{188}Re$, $^{212}Pb$, $^{213}Bi$, $^{211}At$, $^{223}Ra$, and $^{225}Ac$. In some embodiments, the radionuclide comprises or consists of at least one selected from a group consisting of $^{68}Ga$ and $^{177}Lu$. In some embodiments, the radionuclide comprises $^{68}Ga$. In some embodiments, the radionuclide comprises $^{177}Lu$.

In some embodiments, the third compound reacts with the first compound to obtain a fourth compound through a click reaction.

In some embodiments, obtaining the first compound comprises: an $R_2$ cyclic peptide is reacted with bicyclo[6.1.0] nonyne(BCN)-$L_2$-*N*-hydroxysuccinimide (NHS) to obtain the first compound. In some embodiments, obtaining the second compound comprises: a linear peptide with or without a protecting group of an $R_1$ cyclic peptide, $L_1$-$CH_2CH_2OH$ with or without a protecting group, or lysine with or without a protecting group is reacted with DO3A*t*Bu-$N_3$ to obtain the second compound. In some embodiments, the treatment is radiotherapy.

In some embodiments, the present disclosure develops a novel albumin binder that can significantly increase tumor uptake without delivering adverse impact on non-tumor organs, thereby improving the therapeutic index.

In order to further elaborate on the technical means and effects adopted by the present disclosure to achieve the predetermined objects, the specific implementations, structures, features and effects of the present disclosure of the present disclosure are described in detail below in conjunction with the accompanying drawings and preferred embodiments

## Examples

The embodiments and comparative examples of the present disclosure are shown in Table 1. Specifically, Example L00 is a comparative example.

[Table 1]

| Example | $L_1$ | $R_1$ | $L_2$ | $R_2$ | $R_3$ | x | $R_4$ | y | $Q_1$ | $Q_2$ | $Q_3$ | $Q_4$ | $Q_5$ |
|---|---|---|---|---|---|---|---|---|---|---|---|---|---|
| L00 | $(PEG)_4$ | SEQ ID NO:1 | $(PEG)_4$ | SEQ ID NO:3 | Formula (L-1) | 3 | -H | - | - | - | - | - | - |
| L11 | $(PEG)_4$ | SEQ ID NO:1 | $(PEG)_4$ | SEQ ID NO:3 | Formula (L-I) | 3 | Formula (III) | 3 | -H | -H | $-CF_3$ | -H | -H |
| L12 | $(PEG)_4$ | SEQ ID NO:1 | $(PEG)_4$ | SEQ ID NO:3 | Formula (L-I) | 3 | Formula (III) | 3 | -H | -H | $-CF_3$ | -F | -H |
| L13 | $(PEG)_4$ | SEQ ID NO:1 | $(PEG)_4$ | SEQ ID NO:3 | Formula (L-I) | 3 | Formula (III) | 3 | -H | -H | $-CF_3$ | -H | -F |
| L14 | $(PEG)_4$ | SEQ ID NO:1 | $(PEG)_4$ | SEQ ID NO:3 | Formula (L-I) | 3 | Formula (III) | 2 | -H | -H | $-CF_3$ | -H | -H |
| 115 | $(PEG)_4$ | SEQ ID NO:1 | $(PEG)_4$ | SEQ ID NO:3 | Formula (L-I) | 3 | Formula (III) | 3 | -H | -H | -I | -H | -H |
| L16 | $(PEG)_4$ | SEQ ID NO:1 | $(PEG)_4$ | SEQ ID NO:4 | Formula (L-I) | 3 | Formula (III) | 3 | -H | -H | $-CF_3$ | -H | -H |
| L17 | $(PEG)_4$ | SEQ ID NO:1 | $(PEG)_4$ | SEQ ID NO:4 | Formula (L-I) | 3 | Formula (III) | 3 | -H | -H | $-CF_3$ | -F | -H |
| L18 | $(PEG)_4$ | SEQ ID NO:1 | $(PEG)_4$ | SEQ ID NO:4 | Formula (L-I) | 3 | Formula (III) | 3 | -H | -H | $-CF_3$ | -H | -F |
| L19 | $(PEG)_4$ | SEQ ID NO:1 | $(PEG)_4$ | SEQ ID NO:5 | Formula (L-I) | 3 | Formula (III) | 3 | -H | -H | $-CF_3$ | -H | -H |
| L20 | $(PEG)_4$ | SEQ ID NO:1 | $(PEG)_4$ | SEQ ID NO:5 | Formula (L-I) | 3 | Formula (III) | 3 | -H | -H | $-CF_3$ | -F | -H |
| L21 | $(PEG)_4$ | SEQ ID NO:1 | $(PEG)_4$ | SEQ ID NO:5 | Formula (L-I) | 3 | Formula (III) | 3 | -H | -H | $-CF_3$ | -H | -F |
| L11-1 | $(PEG)_4$ | SEQ ID NO:1 | $(PEG)_4$ | SEQ ID NO:6 | Formula (L-I) | 3 | Formula (III) | 3 | -H | -H | $-CF_3$ | -H | -H |
| L 12-1 | $(PEG)_4$ | SEQ ID NO:1 | $(PEG)_4$ | SEQ ID NO:6 | Formula (L-I) | 3 | Formula (III) | 3 | -H | -H | $-CF_3$ | -F | -H |

(continued)

| Example | $L_1$ | $R_1$ | $L_2$ | $R_2$ | $R_3$ | x | $R_4$ | y | $Q_1$ | $Q_2$ | $Q_3$ | $Q_4$ | $Q_5$ |
|---|---|---|---|---|---|---|---|---|---|---|---|---|---|
| L13-1 | $(PEG)_4$ | SEQ ID NO:1 | $(PEG)_4$ | SEQ ID NO:6 | Formula (L-I) | 3 | Formula (III) | 3 | -H | -H | $-CF_3$ | -H | -F |
| D-L11 | $(PEG)_4$ | SEQ ID NO:1 | $(PEG)_4$ | SEQ ID NO:3 | Formula (D-I) | 3 | Formula (III) | 3 | -H | -H | $-CF_3$ | -H | -H |
| D-L 12 | $(PEG)_4$ | SEQ ID NO:1 | $(PEG)_4$ | SEQ ID NO:3 | Formula (D-I) | 3 | Formula (III) | 3 | -H | -H | $-CF_3$ | -F | -H |
| D-L13 | $(PEG)_4$ | SEQ ID NO:1 | $(PEG)_4$ | SEQ ID NO:3 | Formula (D-I) | 3 | Formula (III) | 3 | -H | -H | $-CF_3$ | -H | -F |
| D-L11-1 | $(PEG)_4$ | SEQ ID NO:1 | $(PEG)_4$ | SEQ ID NO:6 | Formula (D-I) | 3 | Formula (III) | 3 | -H | -H | $-CF_3$ | -H | -H |
| D-L12-1 | $(PEG)_4$ | SEQ ID NO:1 | $(PEG)_4$ | SEQ ID NO:6 | Formula (D-I) | 3 | Formula (III) | 3 | -H | -H | $-CF_3$ | -F | -H |
| D-L13-1 | $(PEG)_4$ | SEQ ID NO:1 | $(PEG)_4$ | SEQ ID NO:6 | Formula (D-I) | 3 | Formula (III) | 3 | -H | -H | $-CF_3$ | -H | -F |
| L11-2 | $(PEG)_4$ | SEQ ID NO:2 | $(PEG)_4$ | SEQ ID NO:3 | Formula (L-I) | 3 | Formula (III) | 3 | -H | -H | $-CF_3$ | -H | -H |
| L12-2 | $(PEG)_4$ | SEQ ID NO:2 | $(PEG)_4$ | SEQ ID NO:3 | Formula (L-I) | 3 | Formula (III) | 3 | -H | -H | $-CF_3$ | -F | -H |
| L13-2 | $(PEG)_4$ | SEQ ID NO:2 | $(PEG)_4$ | SEQ ID NO:3 | Formula (L-I) | 3 | Formula (III) | 3 | -H | -H | $-CF_3$ | -H | -F |
| L11-1-2 | $(PEG)_4$ | SEQ ID NO:2 | $(PEG)_4$ | SEQ ID NO:6 | Formula (L-I) | 3 | Formula (III) | 3 | -H | -H | $-CF_3$ | -H | -H |
| L12-1-2 | $(PEG)_4$ | SEQ ID NO:2 | $(PEG)_4$ | SEQ ID NO:6 | Formula (L-I) | 3 | Formula (III) | 3 | -H | -H | $-CF_3$ | -F | -H |
| L13-1-2 | $(PEG)_4$ | SEQ ID NO:2 | $(PEG)_4$ | SEQ ID NO:6 | Formula (L-I) | 3 | Formula (III) | 3 | -H | -H | $-CF_3$ | -H | -F |
| L31 | $(PEG)_4$ | SEQ ID NO:1 | $(PEG)_4$ | SEQ ID NO:3 | Formula (L-II) | 3 | Formula (III) | 3 | -H | -H | $-CF_3$ | -H | -H |
| L32 | $(PEG)_4$ | SEQ ID NO:1 | $(PEG)_4$ | SEQ ID NO:3 | Formula (L-II) | 3 | Formula (III) | 3 | -H | -H | $-CF_3$ | -F | -H |
| L33 | $(PEG)_4$ | SEQ ID NO:1 | $(PEG)_4$ | SEQ ID NO:3 | Formula (L-II) | 3 | Formula (III) | 3 | -H | -H | $-CF_3$ | -H | -F |

(continued)

| Example | $L_1$ | $R_1$ | $L_2$ | $R_2$ | $R_3$ | x | $R_4$ | y | $Q_1$ | $Q_2$ | $Q_3$ | $Q_4$ | $Q_5$ |
|---|---|---|---|---|---|---|---|---|---|---|---|---|---|
| L31-1 | $(PEG)_4$ | SEQ ID NO:1 | $(PEG)_4$ | SEQ ID NO:6 | Formula (L-II) | 3 | Formula (III) | 3 | -H | -H | $-CF_3$ | -H | -H |
| L32-1 | $(PEG)_4$ | SEQ ID NO:1 | $(PEG)_4$ | SEQ ID NO:6 | Formula (L-II) | 3 | Formula (III) | 3 | -H | -H | $-CF_3$ | -F | -H |
| L33-1 | $(PEG)_4$ | SEQ ID NO:1 | $(PEG)_4$ | SEQ ID NO:6 | Formula (L-II) | 3 | Formula (III) | 3 | -H | -H | $-CF_3$ | -H | -F |
| D-L31 | $(PEG)_4$ | SEQ ID NO:1 | $(PEG)_4$ | SEQ ID NO:3 | Formula (D-II) | 3 | Formula (III) | 3 | -H | -H | $-CF_3$ | -H | -H |
| D-L32 | $(PEG)_4$ | SEQ ID NO:1 | $(PEG)_4$ | SEQ ID NO:3 | Formula (D-II) | 3 | Formula (III) | 3 | -H | -H | $-CF_3$ | -F | -H |
| D-L 33 | $(PEG)_4$ | SEQ ID NO:1 | $(PEG)_4$ | SEQ ID NO:3 | Formula (D-II) | 3 | Formula (III) | 3 | -H | -H | $-CF_3$ | -H | -F |
| D-L31-1 | $(PEG)_4$ | SEQ ID NO:1 | $(PEG)_4$ | SEQ ID NO:6 | Formula (D-II) | 3 | Formula (III) | 3 | -H | -H | $-CF_3$ | -H | -H |
| D-L32-1 | $(PEG)_4$ | SEQ ID NO:1 | $(PEG)_4$ | SEQ ID NO:6 | Formula (D-II) | 3 | Formula (III) | 3 | -H | -H | $-CF_3$ | -F | -H |
| D-L33-1 | $(PEG)_4$ | SEQ ID NO:1 | $(PEG)_4$ | SEQ ID NO:6 | Formula (D-II) | 3 | Formula (III) | 3 | -H | -H | $-CF_3$ | -H | -F |

SEQ ID NO: 1-6 stated above are shown in Table 2.

[Table 2]

| SEQ ID NO | Sequence |
|---|---|
| 1 | c(RGDfK) |
| 2 | c(RGDyK) |
| 3 | c(KYNGRT) |
| 4 | c(KGN*Sar*RG] |
| 5 | c(KNGRE) |
| 6 | c(KYN*Sar*RT) |
| Wherein, "c(......)" denotes a cyclic peptide, lowercase roman letters denote D-amino acids, and italicized "*Sar*" denotes sarcosine. | |

*Preparation of Compounds of Examples*

Hereinafter, the compound of Example L11 is taken as an example, but is not limited thereto. Those skilled in the art will understand that the compounds in other examples, including but not limited to the compounds in the examples shown in Table 1, can be synthesized in a manner similar to the synthesis of compound of Example L11 described below.

In the present disclosure, Example L11 may be referred to as "L11," and other examples are designated

accordingly.

1. Preparation of Precursor $R_2$-$L_2$-BCN

1.1 46 µL of *N,N*-diisopropylethylamine (DIEA) (278 µmol) was added to 100 µL of *N,N*-dimethylformamide (DMF) containing 20 mg of cyclic peptide of SEQ ID NO: 3 (27.78 µmol) and 22.5 mg of BCN-$L_2$-NHS ester (41.7 µmol) to obtain a reaction mixture.
1.2 The reaction mixture was stirred at room temperature for 2 h.
1.3 Separation of product: The product was separated by high-performance liquid chromatography (HPLC), with a semi-preparative C18 column as a stationary phase and a gradient elution method as a mobile phase at a flow rate of 4 mL/min, changing from 5% acetonitrile to 50% acetonitrile in 20 min.
1.4 Identification of product: The product was further identified by the HPLC and mass spectrometry.
1.5 Results of electrospray ionization mass spectrometry (ESI-MS): *m*/*z* $[M+H]^+$ = 1144.87 (chemical formula: $C_{53}H_{82}N_{12}O_{16}$, and calculated molecular weight: 1142.60). The electrospray ionization mass spectrum is shown in FIG. 1, wherein the horizontal axis represents mass-to-charge ratio (*m*/*z*), and the vertical axis represents relative intensity (%).

2. Preparation of Precursor $R_1$-$L_1$-Lys-DOTA-$N_3$

2.1 Linear peptide Lys(Dde)-D-Phe-Asp(Otbu)-Gly-Arg(Pbf) (*i.e.* the linear peptide of the protected cyclic peptide of SEQ ID NO: 1) was synthesized by solid-phase synthesis, and the polypeptide thereof was kept linked to a resin.
2.2 Dde protecting group of the side chain of the first lysine was removed with 2% hydrazine hydrate in DMF.
2.3 After the resin was washed with DMF, an Fmoc protecting group of the last arginine was removed by a 20% piperidine in DMF.
2.4 After the resin was washed with DMF, Fmoc-$L_1$-$CH_2CH_2OH$, Fmoc-Lys(Boc)-OH and DO3A*t*Bu-$N_3$ were connected sequentially by solid-phase synthesis.
2.5 After the resin was washed with DMF, the resin was cleaved using a solution of 30% trifluoroethanol in dichloromethane for 2 h, filtering was performed, and the solvent therein was removed using a rotary to obtain a product with a protecting group.
2.6 The product obtained from the previous step was dissolved in dichloromethane, two equivalents of benzotriazole-1-yl-oxytripyrrolidylphosphonium hexafluorophosphate (PyBop) and ten equivalents of DIEA were added, a reaction was performed at reflux of 45°C overnight, and the solvent was removed using a rotary evaporator to obtain a cyclized product.
2.7 A protecting group of the product obtained in the previous step was removed using a 95% trifluoroacetic acid (TFA) cleavage solution.
2.8 The cleavage solution was blown and dried with nitrogen as much as possible, ethyl ether was then added to precipitate a product, a supernatant was removed through centrifugation to obtain a precipitate, and the precipitate was washed with ethyl ether six times, and then evaporated and dried at room temperature.
2.9 Separation of product: The product was separated using HPLC, with a semi-preparative C18 column as a stationary phase and a gradient elution method as a mobile phase at a flow rate of 4 mL/min, changing from 5% acetonitrile to 50% acetonitrile in 20 min.
2.10 Identification of product: The product was further identified by the HPLC and mass spectrometry.
2.11 Results of electrospray ionization mass spectrometry (ESI-MS): *m*/*z*$[M+H]^+$=1434.96 (chemical formula: $C_{62}H_{103}N_{19}O_{20}$, and calculated molecular weight: 1433.76). The electrospray ionization mass spectrum is shown in FIG. 2, wherein the horizontal axis represents a mass-to-charge ratio (*m*/*z*), and the vertical axis represents a relative intensity (%).

3. Preparation of Precursor $R_1$-$L_1$-Lys(-$R_3$-H) -DOTA-$N_3$

3.1 20 mg (13.94 µmol) of $R_1$-$L_1$-Lys-DOTA-$N_3$ was weighed and dissolved in 100 µL of DMF.
3.2 23 µL of DIEA (139.4 µmol) and 11.5 mg of compound of Formula (I') with Boc-protected -N*$H_2$ group (20.89 µmol) were added in sequence to the solution obtained in the previous step to obtain a reaction mixture.
3.3 The reaction mixture was stirred at room temperature for 2 h.
3.4 The product obtained in the previous step was precipitated with ice ethyl ether.
3.5 A protecting group on the -R group of the product obtained in the previous step was removed using a 95% TFA cleavage solution.
3.6 The cleavage solution was blown and dried with nitrogen as much as possible, ethyl ether was then added to precipitate a product, a supernatant was removed through centrifugation to obtain a precipitate, and the precipitate was washed with ethyl ether six times, and then evaporated and dried at room temperature.

3.7 Separation of product: The product was separated using HPLC, with a semi-preparative C18 column as a stationary phase and a gradient elution method as a mobile phase at a flow rate of 4 mL/min, changing from 5% acetonitrile to 50% acetonitrile in 20 min.

3.8 Identification of product: The product was further identified by the HPLC and mass spectrometry.

3.9 Results of electrospray ionization mass spectrometry (ESI-MS): *m*/*z*$[M+2H]^+$=832.25 (chemical formula: $C_{72}H_{119}N_{21}O_{24}$, calculated molecular weight 1661.87). The electrospray ionization mass spectrum is shown in FIG. 3, wherein the horizontal axis represents a mass-to-charge ratio (*m*/*z*), and the vertical axis represents a relative intensity (%).

4. Preparation of Precursor $R_1$-$L_1$-Lys(-$R_3$-H) -DOTA-click-$L_2$-$R_2$

4.1 15 mg (9 μmol) of $R_1$-$L_1$-Lys(-$R_3$-H)-DOTA-$N_3$ and 15 mg (13.14 μmol) of $R_2$-$L_2$-BCN were weighed, respectively, and dissolved in 100μL of DMF to obtain a reaction mixture.

4.2 The reaction mixture was stirred at room temperature for 2 h.

4.3 Separation of product: The product was separated using HPLC, with a semi-preparative C18 column as a stationary phase and a gradient elution method as a mobile phase at a flow rate of 4 mL/min, changing from 5% acetonitrile to 50% acetonitrile in 20 min.

4.4 Identification of product: The product was further identified by the HPLC and mass spectrometry.

4.5 Results of electrospray ionization mass spectrometry (ESI-MS): *m*/*z* $[M+2H]^+$ = 1404.47 (chemical formula: $C_{125}H_{201}N_{33}O_{40}$, and calculated molecular weight: 2804.47). The electrospray ionization mass spectrum is shown in FIG. 4, wherein the horizontal axis represents a mass-to-charge ratio (*m*/*z*), and the vertical axis represents a relative intensity (%).

5. Preparation of $R_1$-$L_1$-Lys(-$R_3$-$R_4$) -DOTA-click-$L_2$-$R_2$ conjugate

5.1 12 mg (4.28 μmol) of $R_1$-$L_1$-Lys(-$R_3$-H) -DOTA-click-$L_2$-$R_2$ were weighed and dissolved in 100 μL of DMF to obtain a solution.

5.2 7 μL of DIEA (42.8 μmol) and 5.6 mg of a compound of Formula (III') (17 μmol) were added in sequence to the solution obtained in the previous step to obtain a reaction mixture.

5.3 The reaction mixture was stirred at room temperature for 2 h.

5.4 1M of a TFA aqueous solution was added to the reaction mixture, and pH of the reaction solution was adjusted to 5.0-6.0.

5.5 Separation of product: The product was separated using HPLC, with a semi-preparative C18 column as a stationary phase and a gradient elution method as a mobile phase at a flow rate of 4 mL/min, changing from 20% acetonitrile to 32% acetonitrile in 20 min.

5.6 Identification of product: The product was further identified by the HPLC and mass spectrometry.

5.7 Results of electrospray ionization mass spectrometry (ESI-MS): *m*/*z* $[M+2H]^+$ = 1510.58 (chemical formula: $C_{136}H_{210}F_3N_{33}O_{41}$, and calculated molecular weight: 3018.53). The electrospray ionization mass spectrum is shown in FIG. 5, wherein the horizontal axis represents a mass-to-charge ratio (*m*/*z*), and the vertical axis represents a relative intensity (%).

*Stability Study*

The experiment was carried out by using an *in vitro* incubation method at a constant temperature (37°C) to study the stability of the compound (1.0 μg/mL) from Example L11 in murine plasma or PBS. The compound obtained from Example L11 was dissolved in 1 mL of mouse serum, respectively, and incubated *in vitro* at a constant temperature for 0 h, 0.5 h, 1 h, 4 h, 24 h, and 48 h, and a percentage of drug remaining was then measured and determined. PBS was used as a negative control group. Peak areas of the drug and an internal standard were measured using an LC-MS/MS method, and ratios of the peaks were used instead of the drug concentration for calculation.

Results were shown in Table 3.

Table 3 Percentage of drug remaining of cyclic peptides of the compound of Example L11 after *in vitro* incubation at constant temperature in murine plasma or PBS

| System | Percentage of drug remaining(%) | | | | | |
|---|---|---|---|---|---|---|
| | 0 h | 0.5 h | 1 h | 4 h | 24 h | 48 h |
| murine plasma | 100 | 98.54 | 98.39 | 98.03 | 93.38 | 86.45 |

(continued)

| System | Percentage of drug remaining(%) | | | | | |
|---|---|---|---|---|---|---|
| | 0 h | 0.5 h | 1 h | 4 h | 24 h | 48 h |
| PBS | 100 | 99.27 | 98.69 | 98.61 | 98.35 | 97.89 |

It can be seen from the table above that the percentage of drug remaining of cyclic peptides of the compound of Example L11 after *in vitro* incubation at constant temperature in murine plasma or PBS was basically maintained above 95%, indicating a high stability.

*Animal Experiments*

1. Modeling of human pancreatic cancer xenograft

BxPC3 human pancreatic cancer cells are suspended in a mixture of 200 ¡.tL phosphate buffer and matrix gel (v/v, 1/1), and $2\times10^6$ cells were implanted subcutaneously into left shoulders of normal NCr nude mice (18-25 g, 4-6 weeks old). After an average of 1.5 weeks, a tumor diameter reached about 10 mm, which was sufficient for biodistribution and single-photon emission computed tomography-computed tomography (SPECT/CT) imaging studies.

2. $^{68}$Ga labeling of compound

2.1 1.0 μmol of the compound from Example L11 was dissolved in 1.0 mL of 0.25 M sodium acetate solution.

2.2 4 mL of 0.05 M hydrochloric acid solution was used to elute a germanium-gallium generator to prepare a $^{68}GaCl_3$ eluent.

2.3 1 μmol of the compound from Example L11 was taken and added to an eluent with 1 mCi activity, 300 μL of 0.25 M sodium acetate solution was then added to allow for reaction at 60°C for 10 min to obtain a $^{68}$Ga-L11 complex. A resulting mixture was monitored and quantitatively labeled by radio-HPLC, with a purity being >97%.

3. Positron emission tomography/computed tomography (PET/CT) imaging of small animals

PET-CT and image analysis were performed using a small-animal NovelMedcal PET-CT scanner (Beijing Yongxin). Maximum tangential and radial half-widths of the scanner in a center of field of view were 1.5 mm, and at an edge of the field of view were 1.8 mm.

About 100 μCi of each of the following complexes: $^{68}$Ga-L00, $^{68}$Ga-L11, $^{68}$Ga-L12, $^{68}$Ga-L13, and $^{68}$Ga-L21, was injected into BxPC3 tumor-bearing mice via tail vein under isoflurane anesthesia, and each of the complexes was injected into four mice. Static PET-CT images were obtained for 15 min 0.5, 1, 2, 3, 4, 5 and 6 hours after intravenous injection. Distribution patterns of the five complexes at each time point were compared.

PET and CT images were acquired using NMSoft workstation software (Beijing Yongxin), and data were given as a percentage of injected dose per gram of tissue or organ (ID/g), and were determined through decay correction for each sample (standardized to a known weight of the injected dose. Normalization was performed during statistical analysis, and standardized uptake values (SUVs) were calculated according to the following formula:

$$SUV= ([Bq/mL] \times [Animal\ weight\ (g)]/ [Injected\ dose\ (Bq)]$$

Uptake of the five complexes in tumors, muscle, and kidneys at each time point was shown in FIGs. 6 and 7.

For the complex $^{68}$Ga-L00 formed from the conjugate of Example L00 without containing an albumin-binding fragment in a conjugate structure, the drug was rapidly metabolized through the kidneys within 2 h after injection, and signals in the tumor tissue gradually weakened. A tumor-to-kidney ratio after 2 h was only approximately 0.18.

After the injection of the remaining four complexes, signals were detectable in systemic blood pools of the mice in a short period of time, indicating that albumin-binding fragments in the complexes had functioned effectively, and increasing circulation time of the complexes in vivo. Moreover, as time went by, most of the complexes were excreted through the kidneys via urine, and background signals in normal tissues throughout the body gradually decreased, while the signals in tumor tissues gradually increased, indicating that the complexes exhibited specific accumulation in tumor tissues over time.

Specifically, for the complex $^{68}$Ga-L 11 formed from by the compound obtained from Example L11, a tumor-to-kidney ratio reached approximately 1.4 about 6 hours after the injection, with a trend of continued increase, indicating that

the compound from Example L11 exhibited good stability, targeting ability, and specificity.

After injection of the complex $^{68}$Ga-L12, an overall imaging trend was similar to that of the $^{68}$Ga-L11 complex, but the accumulation in tumor tissues after 5-6 hours was not as obvious as that of the complex $^{68}$Ga-L11.

The complex $^{68}$Ga-L13 also showed a similar trend, but an overall uptake in tumor tissues was lower than that of the complex $^{68}$Ga-L11.

For the complex $^{68}$Ga-L21, since key peptide targeted head and albumin-binding fragment in a conjugate of Example L21 were identical to those in a conjugate of Example L21, differing only in one linker structure, trend and degree of tissue distribution the two were very similar.

FIG. 7A showed a representative animal from each of the complex groups to illustrate in vivo distribution of the drug 5 hours after injection.

Quantitative results from MIP images and ROI regions of tumors, kidneys, and muscles showed that tumor-to-kidney ratio and tumor-to-muscle ratio of the complex $^{68}$Ga-L11 were highest among the five conjugates. However, compared to the complex $^{68}$Ga-L00, in vivo stability and tumor tissue accumulation of the other four complexes were significantly improved.

4. $^{177}$Lu labeling of compound of Example L11

6 μL of a 0.5 nmol/μL solution of the compound of Example L11 was added to 1 mCi of $^{177}LuCl_3$ to obtain a mixture, with a buffer being 200 μL of 0.1 M acetic acid-ammonium acetate buffer (pH = 3.7-4.0), and the mixture was heated at 90°C for 15 min.

A resulting mixture was monitored and quantitatively labeled by radio-HPLC (>97%) to obtain complex $^{177}$Lu-L11.

5. Single-photon emission computed tomography-computed tomography (SPECT-CT) imaging of small animals

SPECT-CT and image analysis were performed using a small-animal NovelMedical SPECT-CT scanner (Novel Medical Equipment Ltd., Beijing). Maximum tangential and radial half-widths of the scanner in a center of field of view were 1.5 mm, and at an edge of the field of view were 1.8 mm.

About 500 μCi of complex $^{177}$Lu-L11 was injected into BxPC3 tumor-bearing mice via tail vein under isoflurane anesthesia, with a total of 4 animals injected. Static SPECT-CT images were obtained for 30 min 4, 24, 48, 72 and 96 hours after intravenous injection. The long-term effect of complex $^{177}$Lu-L11 was evaluated.

SPECT-CT imaging results were shown in FIG. 8. Radiographic signals were continuously detected in the tumor tissues of the mice within 4 days after intravenous injection of the $^{177}$Lu-L11 solution, while no signals were detected in other tissues and organs, indicating that the compound from Example L11 exhibited good in vivo stability and targeting ability, having the potential for being used for radiotherapy of tumors in combination with the long-half-life therapeutic radionuclide $^{177}$Lu.

6. Biodistribution study

The complexes of $^{177}$Lu-L11, $^{177}$Lu-L12, $^{177}$Lu-$_{D}$L11, and $^{177}$Lu-L11-1 (3.7 MBq) were intravenously injected into BxPC3 tumor-bearing NCr nude mice. Five mice were euthanized at 1, 4, 24, 48, 72, and 120 hours after injection, and then dissected.

Samples of corresponding tissues and organs were then collected, and the collected tissues and organs were weighed, and radioactivity thereof was measured using a γ counter.

FIG. 9 shows distribution of radioactivity in various tissues of mice 24 hours after intravenous injection of the four complexes $^{177}$Lu-L11, $^{177}$Lu-L12, $^{177}$Lu-$_{D}$L11, and 177Lu-L11-1. Vertical axis values denoted a percentage of injected dose per gram of tissue or organ (ID/g), and were determined through decay correction for each sample (standardized to a known weight of the injected dose.

As can be seen from FIG. 9, 24 hours after intravenous injection, the four complexes $^{177}$Lu-L11, $^{177}$Lu-L12, $^{177}$Lu-$_{D}$L11 and $^{177}$Lu-L11-1 were all accumulated in the tumor, with $^{177}$Lu-L12 showing a highest accumulation. In addition, the four complexes also exhibited certain levels of accumulation in the kidneys and low levels of accumulation in the spleen and uterus.

7. Statistics analysis

Statistical analysis was performed using a two-sided unpaired t-test using GraphPad Prism 6. When a probability (p) value was less than 0.05, it was considered statistically significant.

The above embodiments are merely preferred embodiments of the present disclosure and cannot be used to limit

the scope of protection of the present disclosure, and any non-substantial variations and substitutions made by those skilled in the art on the basis of the present disclosure shall fall within the scope of protection claimed by the present disclosure.

## Claims

**1.** A compound comprising a structure in Formula (A),

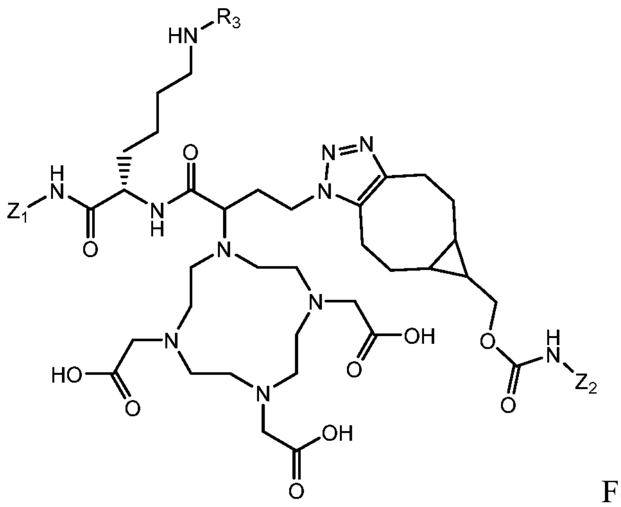

Formula (A),

wherein,

the $Z_1$ is $-L_1R_1$ or -H, the $Z_2$ is $-L_2R_2$ or -H, in the provisos that the $Z_1$ is $-L_1R_1$ and/or the $Z_2$ is $-L_2R_2$;
the $L_1$ is $-(PEG)_m-$, and the *m* is an integer selected from 4 to 30;
the $L_2$ is $-(PEG)_n-$, and the *n* is an integer selected from 4 to 30;
the $R_1$ comprises a peptide sequentially connected by arginine, glycine or sarcosine, and aspartic acid;
the $R_2$ comprises a peptide sequentially connected by asparagine, glycine or sarcosine, cysteine, and arginine;
the $R_3$ is a group of Formula (I) or (II);

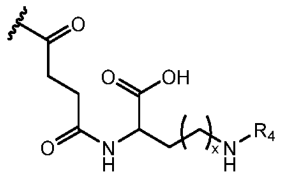

Formula (I),

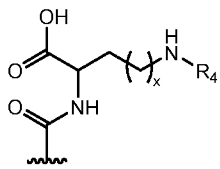

Formula (II);

the $R_4$ is a group of Formula (III), a group of Formula (IV), or a long-chain fatty acid;

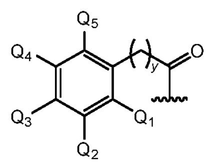

Formula (III),

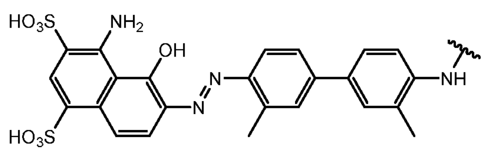

Formula (IV);

the $Q_1$, the $Q_2$, the $Q_3$, the $Q_4$, and the $Q_5$ are each independently selected from a group consisting of -H, -F, -Cl, -Br, -I, $C_1$-$C_6$ linear or branched alkyl, $C_1$-$C_6$ linear or branched fluoroalkyl, and $C_1$-$C_6$ linear or branched fluoroalkoxy;
the *x* is an integer selected from a group consisting of 1, 2, 3, 4, 5, 6, 7, and 8; and
they is an integer selected from a group consisting of 1, 2, 3, 4, 5, and 6.

**2.** The compound according to claim 1, wherein

the $Z_1$ is $-L_1R_1$ and the $Z_2$ is $-L_2R_2$; and/or
the *m* is 4; and/or
the *n is* 4.

**3.** The compound according to claim 1 or 2, wherein

the *x* is 3; and/or
the $R_4$ is a group of Formula (III), and the *y* is 2 or 3.

**4.** The compound according to any one of claims 1-3, wherein

the $R_4$ is a group of Formula (III), and
the $Q_1$ is -H; the $Q_2$ is -H; the $Q_3$ is selected from a group consisting of -H, -F, $-C_1$, -Br, -I, - $CH_2F$, $-CHF_2$, and $-CF_3$; the $Q_4$ is -H or -F; and/or the $Q_5$ is -H or -F;

optionally, the $Q_1$ is -H; the $Q_2$ is -H; the $Q_3$ is $-CF_3$; the $Q_4$ is -H or -F; and/or the $Q_5$ is -H or -F; and
optionally, the $Q_1$ is -H; the $Q_2$ is -H; the $Q_3$ is $-CF_3$; the $Q_4$ is -H or -F; and the $Q_5$ is -H or -F.

**5.** The compound according to any one of claims 1-4, wherein

the $R_1$ is a cyclic peptide and further comprises lysine;

optionally, the $R_1$ is a cyclic peptide, comprising a tripeptide sequence sequentially connected by L-arginine, glycine or sarcosine, and L-aspartic acid; and L-lysine;
optionally, the $R_1$ is a cyclic peptide consisting of 4-7 amino acids;
optionally, the $R_1$ is a cyclic peptide consisting of 5 amino acids;
optionally, the $R_1$ is a cyclic peptide shown in any one of SEQ ID NO: 1-2; and/or

the $R_2$ is a cyclic peptide and further comprises lysine;

optionally, the $R_2$ is a cyclic peptide, comprising a tripeptide sequence sequentially connected by L-asparagine, glycine or sarcosine, and L-arginine; and L-lysine;
optionally, the $R_2$ is a cyclic peptide consisting of 4-7 amino acids;
optionally, the $R_2$ is a cyclic peptide consisting of 5-6 amino acids; and
optionally, the $R_2$ is a cyclic peptide shown in any one of SEQ ID NO: 3-6.

**6.** Use of the compound according to any one of claims 1-5 in radionuclide labeling or preparation of a radionuclide labeling reagent.

**7.** A radionuclide preparation, comprising:

the compound according to any one of claims 1-3; and
a radionuclide, chelated by said compound.

**8.** The radionuclide preparation according to claim 7, wherein the radionuclide comprises or consists of at least one selected from a group consisting of $^{44}Sc$, $^{47}Sc$, $^{62}Cu$, $^{64}Cu$, $^{67}Cu$, $^{66}Ga$, $^{67}Ga$ , $^{68}Ga$ , $^{86}Y$, $^{90}Y$, $^{89}Zr$, $^{99m}Tc$, $^{110m}In$, $^{111}In$, $^{113m}In$, $^{114m}In$, $^{177}Lu$, $^{188}Re$, , $^{203}Pb$, $^{212}Pb$, $^{212}Bi$, $^{213}Bi$, $^{211}At$, $^{223}Ra$, and $^{225}Ac$;

optionally, the radionuclide comprises at least one selected from a group consisting of $^{44}Sc$, $^{47}Sc$, $^{64}Cu$ $^{67}Cu$, $^{67}Ga$, $^{68}Ga$ , $^{90}Y$, $^{99m}Tc$, $^{111}In$, $^{177}Lu$, $^{188}Re$, $^{212}Pb$, $^{213}Bi$, $^{211}At$, $22^{3}Ra$, and $^{225}Ac$; and
optionally, the radionuclide comprises $^{68}Ga$ or $^{177}Lu$.

**9.** A method for preparing the compound according to any one of claims 1-6 or the radionuclide preparation according to claim 7 or 8, comprising:

obtaining a first compound comprising a structure of Formula (A1),

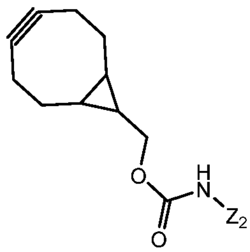
Formula (A1);

obtaining a second compound comprising a structure of Formula (A2),

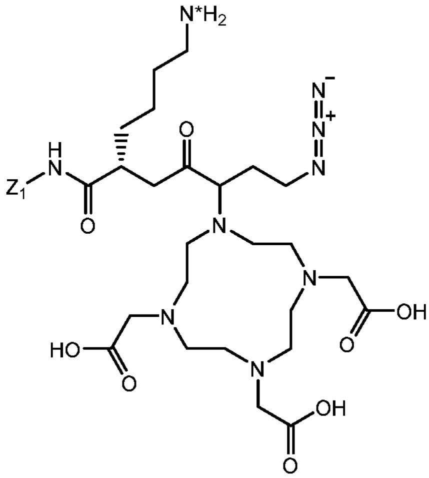
Formula (A2);

forming an amide bond between an -N*$H_2$ group of the second compound and a -C*OOH group of a compound of Formula (I') or Formula (II') or an -N*$H_2$ group protected compound thereof, to obtain a third compound comprising a group of Formula (I") or Formula (II");

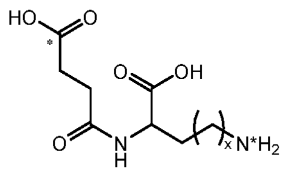
Formula (I'),
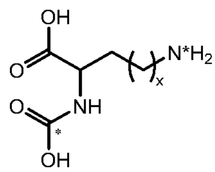
Formula (II'),

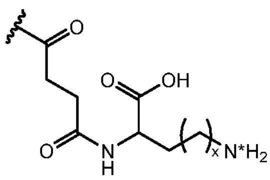
Formula (I"),
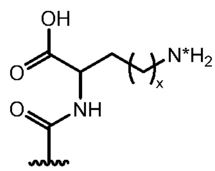
Formula (II");

reacting between the third compound and the first compound to obtain a fourth compound; and
forming an amide bond through an -N*$H_2$ group of a group of Formula (I") or Formula (II") of the fourth compound and a -C*OOH group of a compound of Formula (III'),

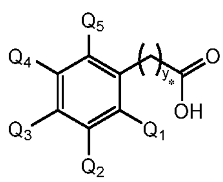
Formula (III').

**10.** Use of the compound according to any one of claims 1-5, or the radionuclide preparation according to any one of

claims 7 or 8 in the preparation of a medicament for detecting cancer, diagnosing cancer, monitoring cancer progression, monitoring cancer treatment, or treating cancer.

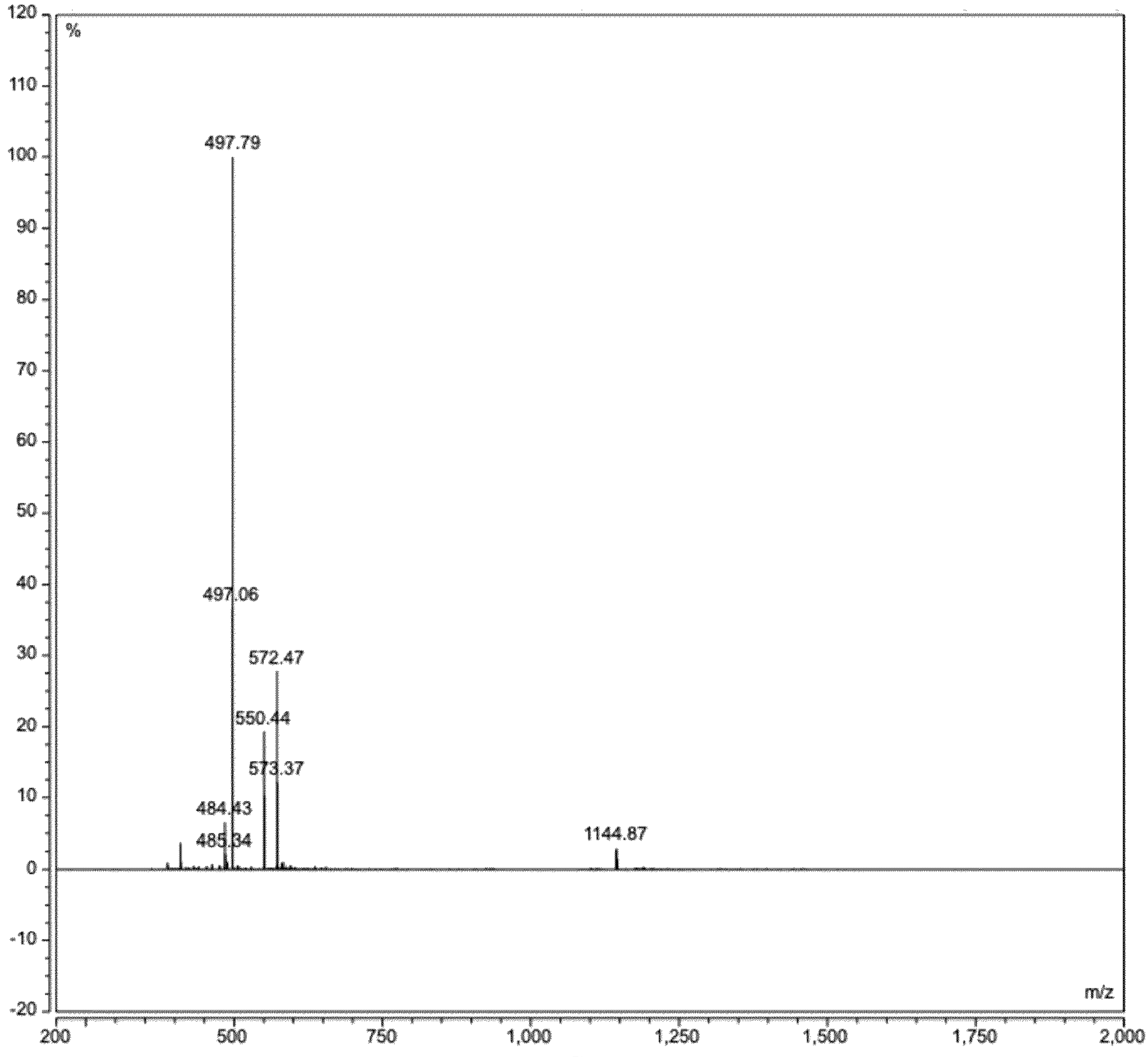
%
120
110
100
90
80
70
60
50
40
30
20
10
0
-10
-20
497.79
497.06
572.47
550.44
573.37
484.43
485.34
1144.87
m/z
200
500
750
1,000
1,250
1,500
1,750
2,000

FIG. 1

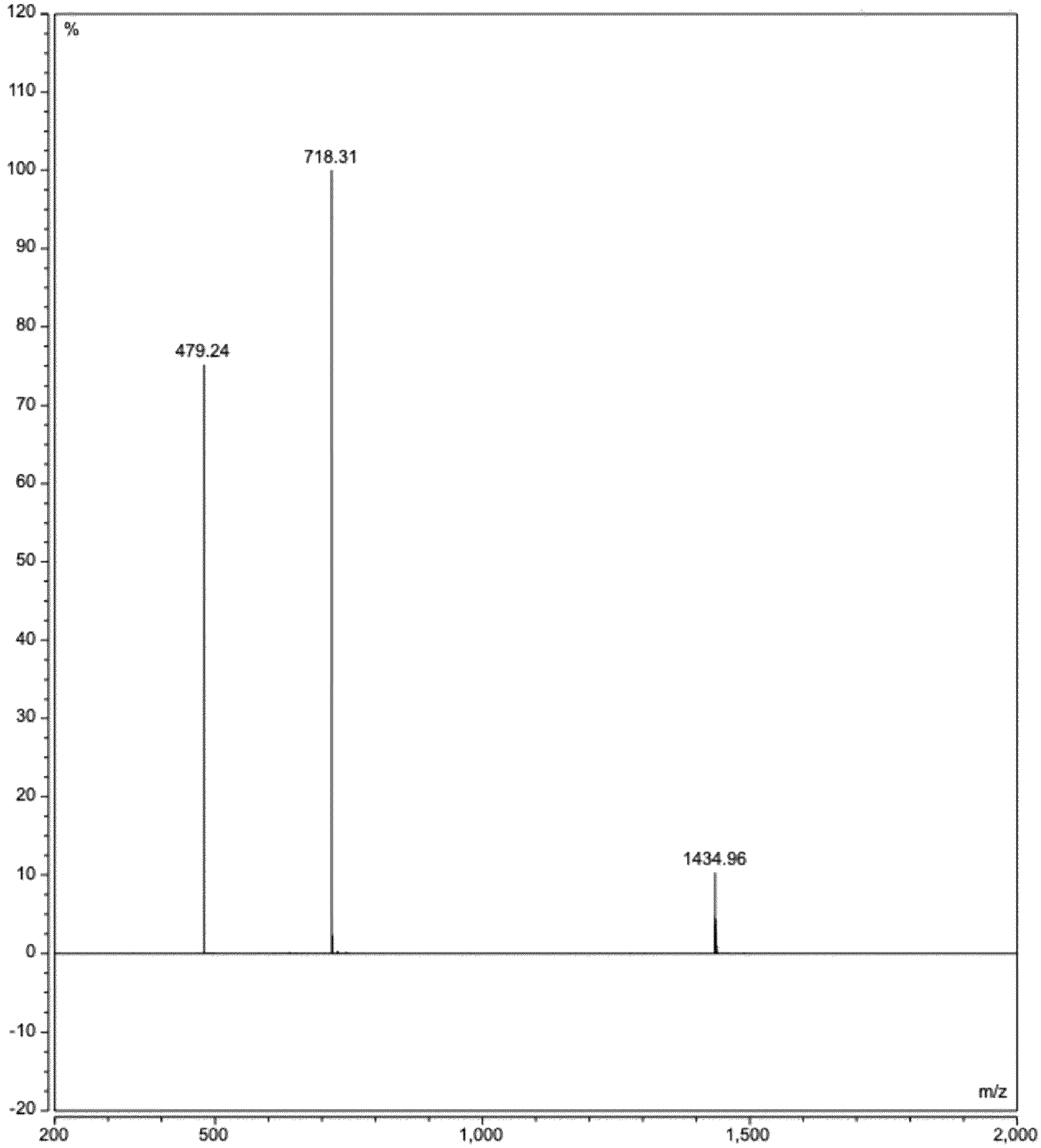
%
120
110
100
90
80
70
60
50
40
30
20
10
0
-10
-20
718.31
479.24
1434.96
m/z
200
500
1,000
1,500
2,000
FIG. 2

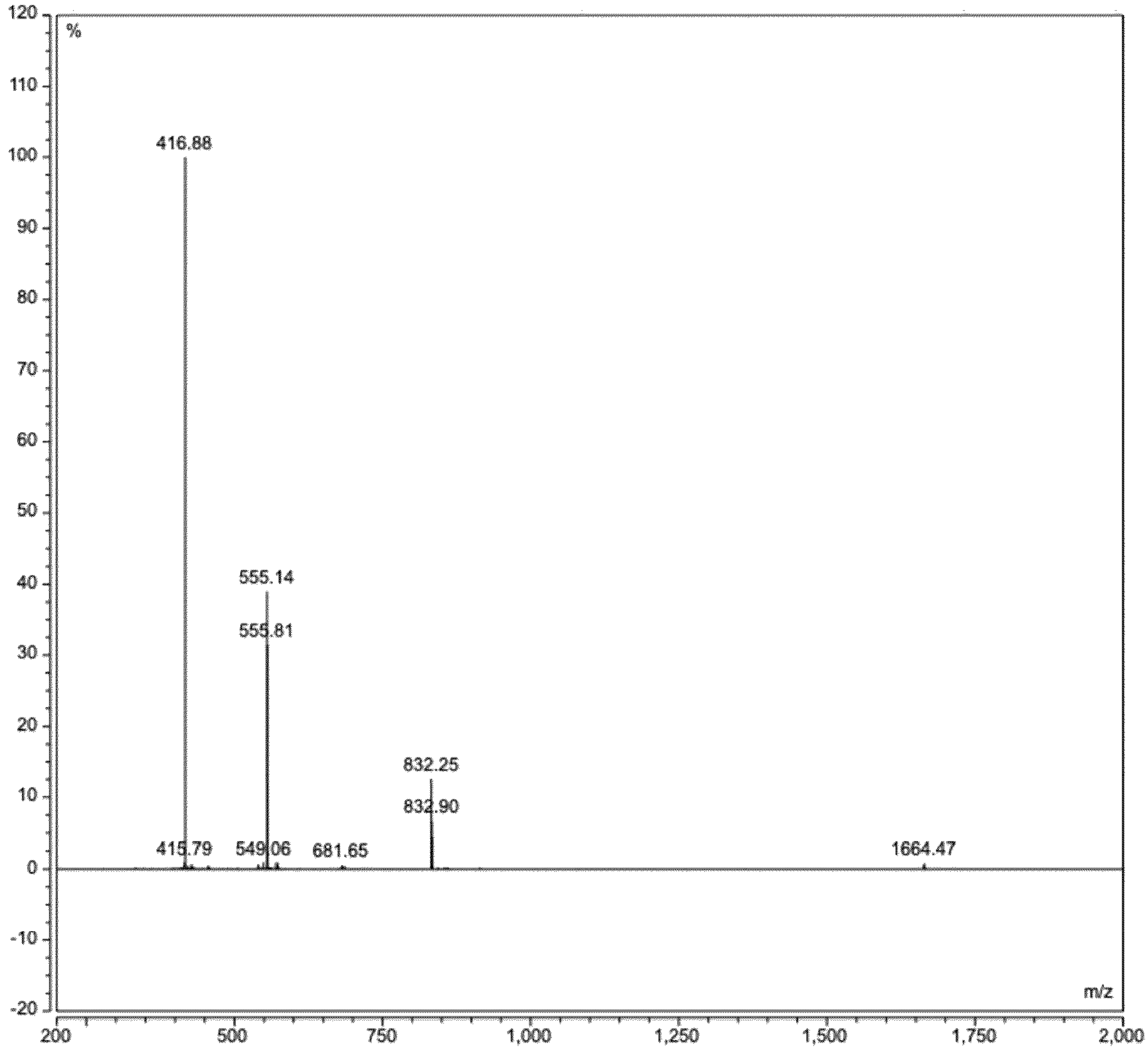
%
120
110
100
90
80
70
60
50
40
30
20
10
0
-10
-20
416.88
555.14
555.81
832.25
832.90
415.79
549.06
681.65
1664.47
m/z
200
500
750
1,000
1,250
1,500
1,750
2,000

FIG. 3

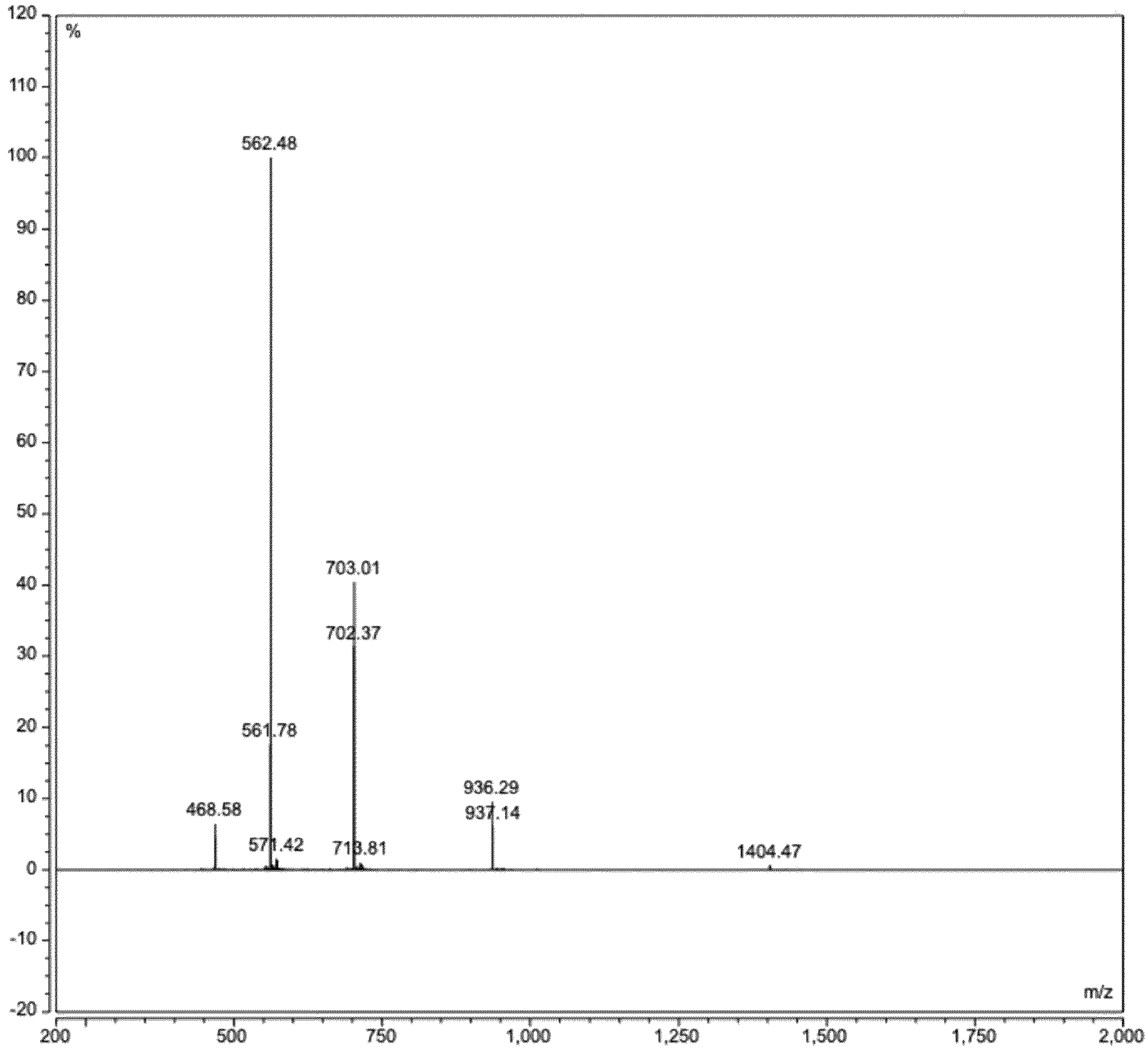
%
562.48
703.01
702.37
561.78
936.29
937.14
468.58
571.42
713.81
1404.47
m/z
120
110
100
90
80
70
60
50
40
30
20
10
0
-10
-20
200
500
750
1,000
1,250
1,500
1,750
2,000
FIG. 4

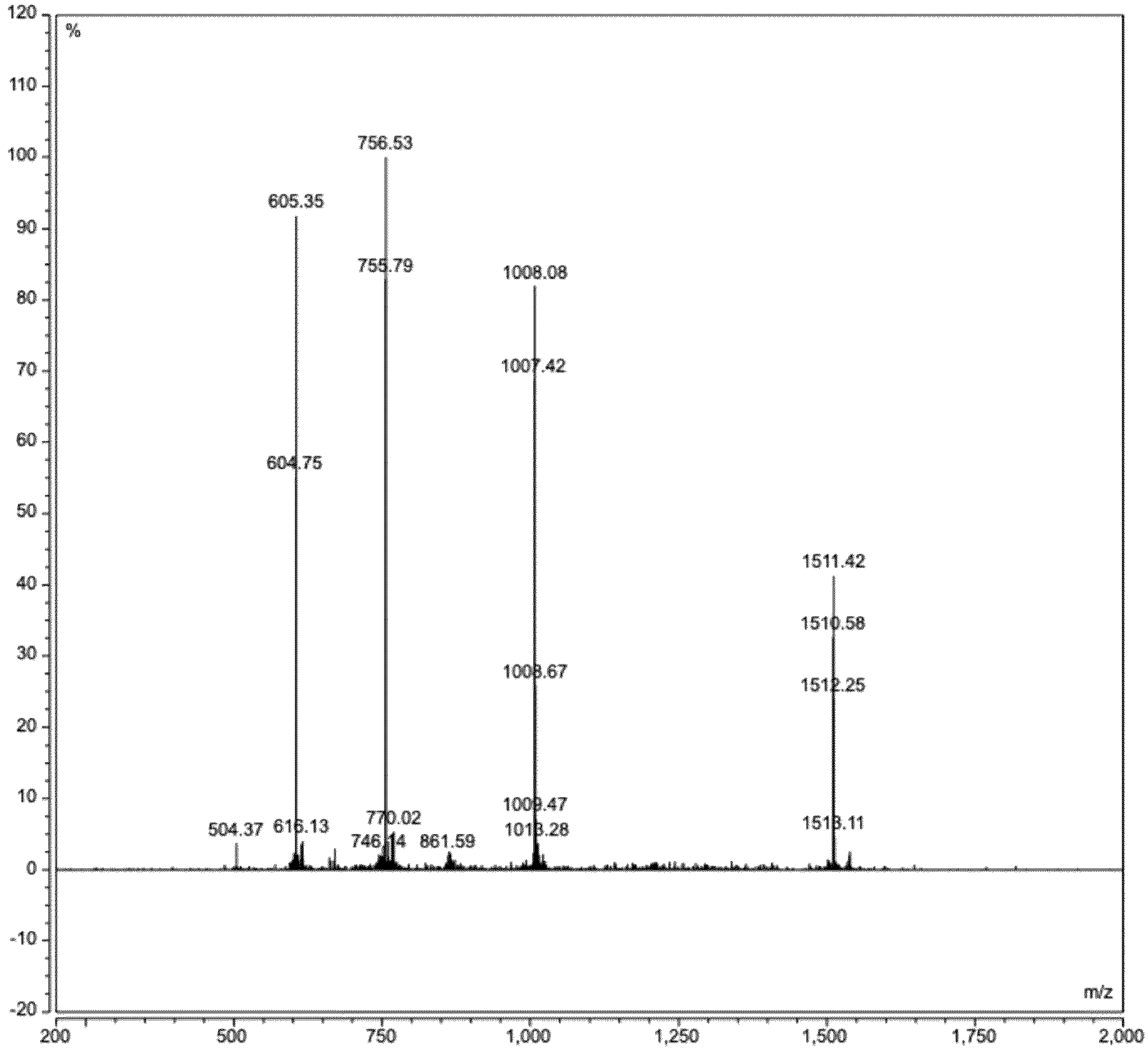
%
120
110
100
90
80
70
60
50
40
30
20
10
0
-10
-20
756.53
605.35
755.79
1008.08
1007.42
604.75
1511.42
1510.58
1008.67
1512.25
1009.47
770.02
504.37
616.13
1513.11
746.14
861.59
1013.28
m/z
200
500
750
1,000
1,250
1,500
1,750
2,000

FIG. 5

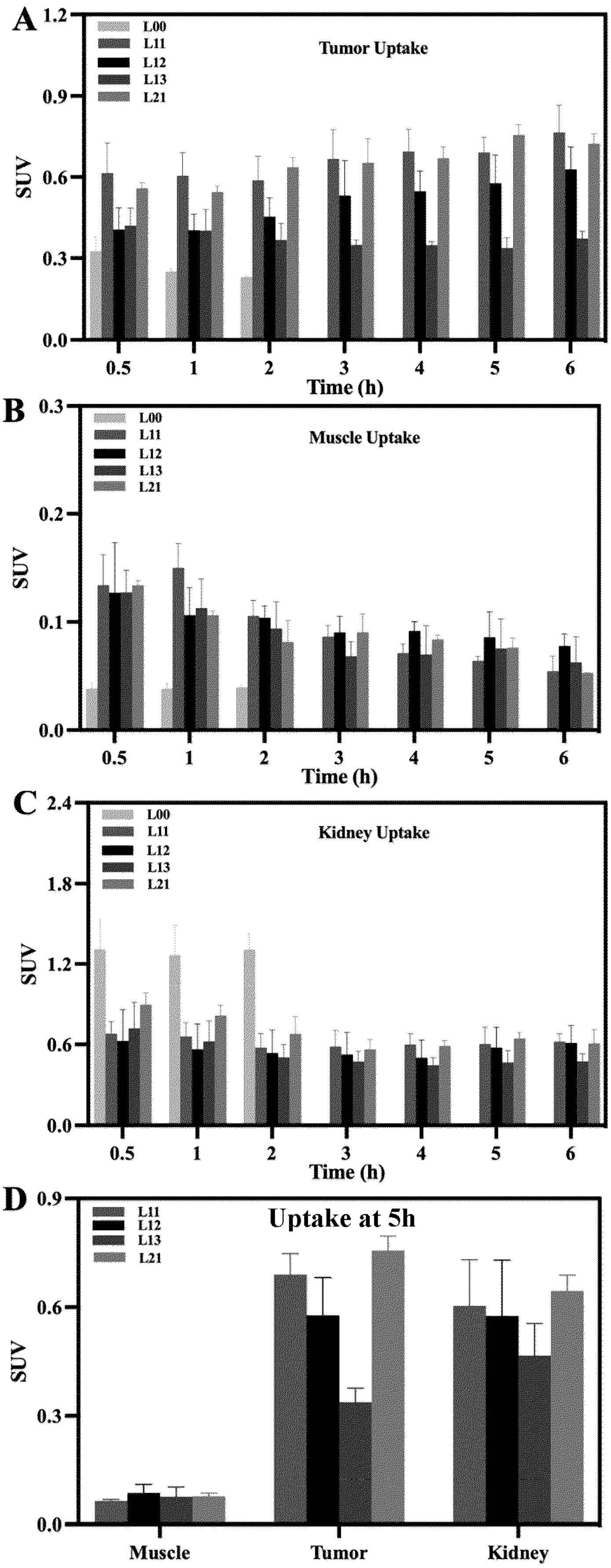
A
Tumor Uptake
L00
L11
L12
L13
L21
SUV
Time (h)
B
Muscle Uptake
C
Kidney Uptake
D
Uptake at 5h
Muscle
Tumor
Kidney

FIG. 6

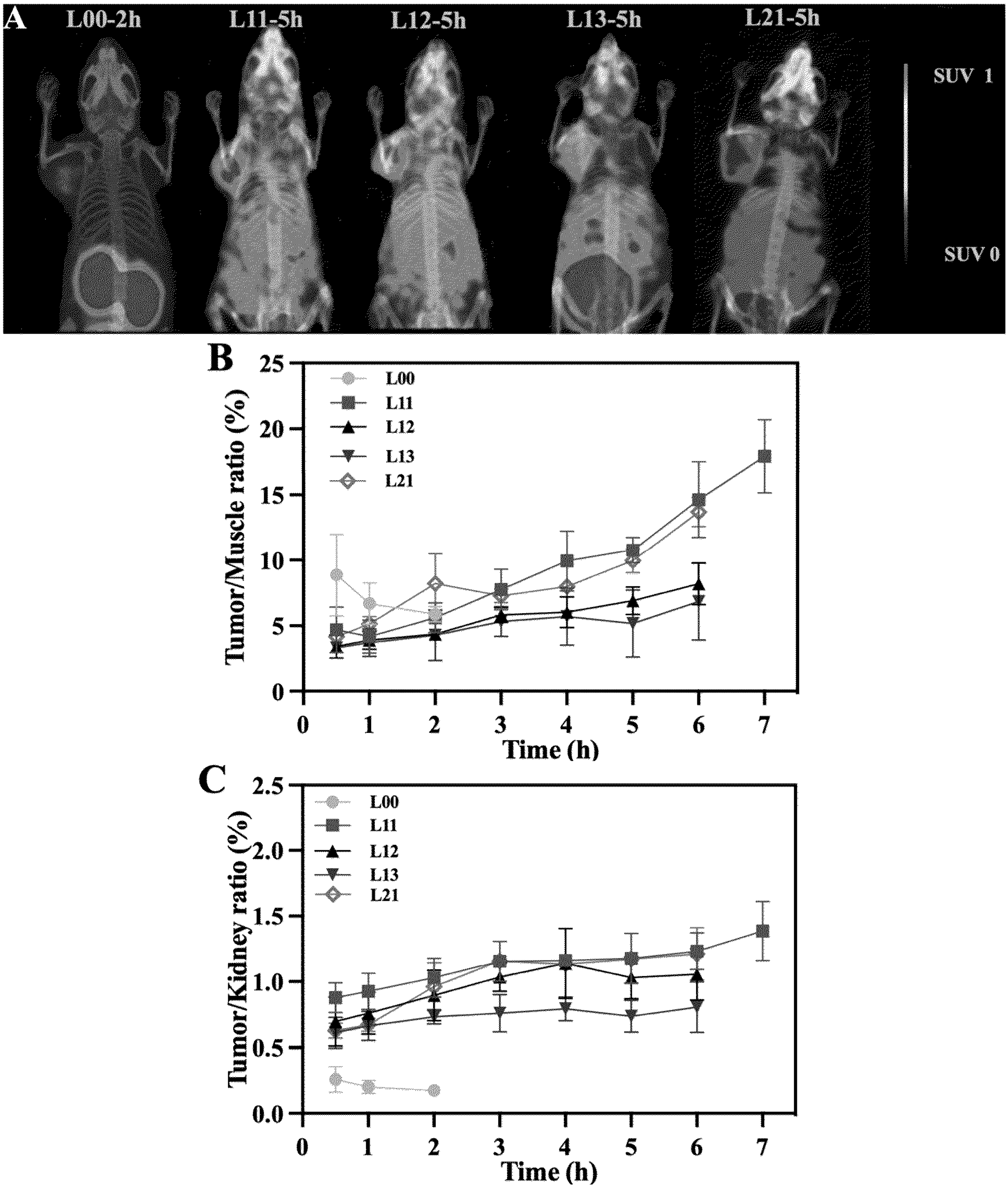
A
L00-2h
L11-5h
L12-5h
L13-5h
L21-5h
SUV 1
SUV 0
B
Tumor/Muscle ratio (%)
L00
L11
L12
L13
L21
Time (h)
C
Tumor/Kidney ratio (%)
L00
L11
L12
L13
L21
Time (h)

FIG. 7

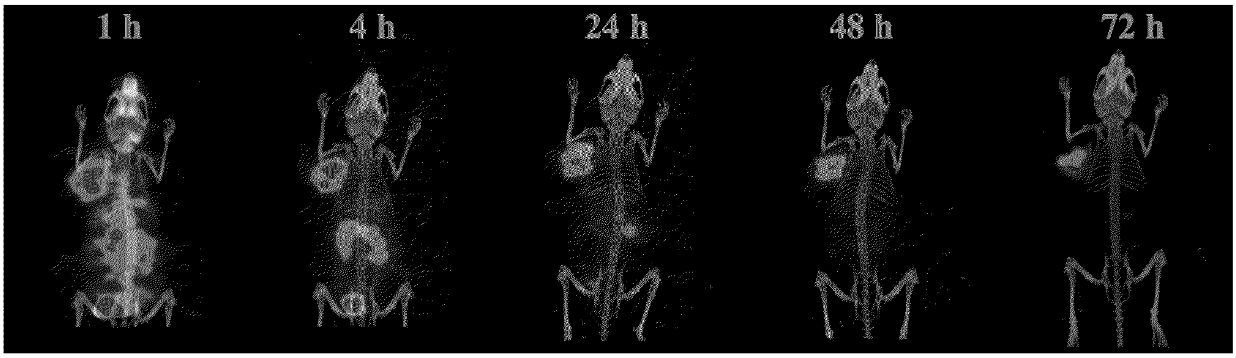

FIG. 8

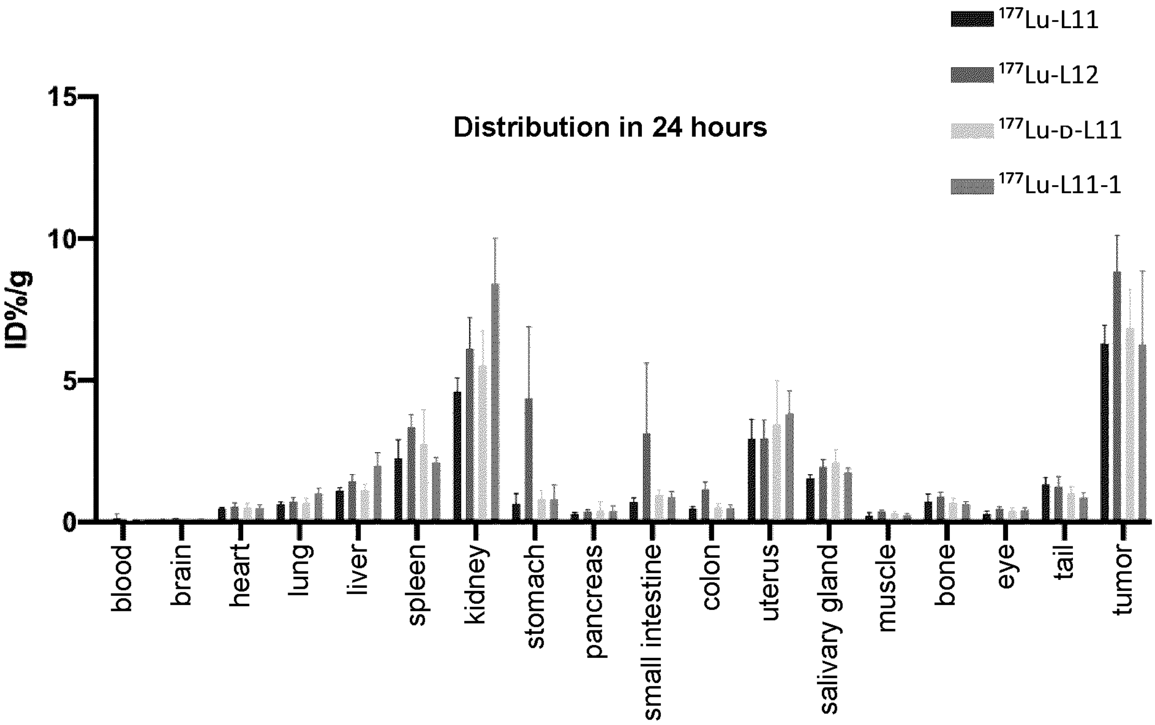

FIG. 9

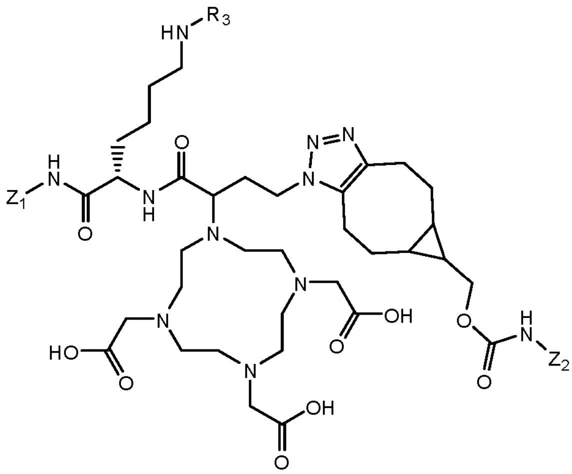
FIG. 10

**INTERNATIONAL SEARCH REPORT**

International application No. **PCT/CN2023/135845**

**A. CLASSIFICATION OF SUBJECT MATTER**

A61K51/08(2006.01)i；A61K51/04(2006.01)i；C07D207/46(2006.01)i；A61K31/16(2006.01)i；A61K31/195(2006.01)i；A61K31/197(2006.01)i；A61K31/197(2006.01)i；A61K31/165(2006.01)i；C07K5/078(2006.01)i

According to International Patent Classification (IPC) or to both national classification and IPC

**B. FIELDS SEARCHED**

Minimum documentation searched (classification system followed by classification symbols)

IPC: A61K,C07D,

Documentation searched other than minimum documentation to the extent that such documents are included in the fields searched

Electronic data base consulted during the international search (name of data base and, where practicable, search terms used)

CNTXT, ENTXT, VCN, DWPI, VEN, 超星读秀学术, DUXIU ACADEMIC, 中国专利生物序列检索系统, China Patents Biological Sequence Search System, CNKI, 万方, WANFANG, ISI web of knowledge, Elsevier Science Direct, Springerlink, pubmed, genbank, ENA, EMBL, Uniport, STN, 百度, BAIDU, 百度学术, BAIDU SCHOLAR, patentics, himmpat: 核欣（苏州）医药科技有限公司, HEXIN (SUZHOU) PHARMACEUTICAL TECHNOLOGY, 单长宇, SHAN CHANGYU, 曾德兴, ZENG DEXING, 陈银飞, CHEN YINFEI, 毛亮, MAO LIANG, 放射性核素, radionuclide, $^{68}Ga$, $^{177}Lu$, 癌症, 肿瘤, tumor, cancer, 螯合剂, chelator , DOTA, 1, 4, 7, 10-四氮杂环十二烷-1, 4, 7, 10-四羧酸, 1, 4, 7, 10-tetraazacyclododecane-N, N', N, N'-tetraacetic acid, 环肽, c(RGDfK), c(RGDyK), c(KYNGRT), c(KGNSarRG), c(KNGRE), c(KYNSarRT), 白蛋白结合剂, click, SEQ ID NOs: 1-6

**C. DOCUMENTS CONSIDERED TO BE RELEVANT**

| Category* | Citation of document, with indication, where appropriate, of the relevant passages | Relevant to claim No. |
|---|---|---|
| Y | CN 113717246 A (HEXIN (SUZHOU) PHARMACEUTICAL TECHNOLOGY CO., LTD.) 30 November 2021 (2021-11-30)<br>claims 1 and 10, description, paragraph 3, and figures 2, 4, and 5 | 1-10 |
| Y | GAI, Y.K. et al. "Evaluation of an Integrin αvβ3 and Aminopeptidase N Dual-Receptor Targeting Tracer for Breast Cancer Imaging"<br>*Molecular Pharmaceutics,* Vol. 17, No. 1, 06 January 2020 (2020-01-06), S13-S21<br>page 349, abstract, page 350, sections 2.1-2.3, and page 352, figure 1 | 1-10 |
| Y | WO 2022040607 A1 (OREGON HEALTH & SCIENCE UNIVERSITY) 24 February 2022 (2022-02-24)<br>abstract, claim 1, description, page 1, line 32 to page 2, line 30, page 3, lines 1-5, page 10, line 5 to page 11, line 5, page 15, lines 16-28, and page 56, lines 5-10, and figures 2-3 | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **01 March 2024** | **06 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2023/135845

C. DOCUMENTS CONSIDERED TO BE RELEVANT

| Category* | Citation of document, with indication, where appropriate, of the relevant passages | Relevant to claim No. |
|---|---|---|
| Y | KILIAN, K. "68Ga-DOTA and Analogs: Current Status and Future Perspectives" *Reports of Practical Oncology and Radiotherapy,* Vol. 19 (supplement), 19 May 2014 (2014-05-19), S13-S21 page S13, abstract, page S15, figure 1, and page S16, left column, paragraph 3 and right column, 2nd-to-last paragraph | 1-10 |
| Y | CN 107207564 A (AMUNIX OPERATING INC.) 26 September 2017 (2017-09-26) description, paragraphs 329-330 | 5-10 |
| A | CN 109416359 A (UNIVERSITY OF PITTSBURGH OF THE COMMONWEALTH SYSTEM OF HIGHER EDUCATION) 01 March 2019 (2019-03-01) abstract, and claims 1-68 | 1-10 |

Form PCT/ISA/210 (second sheet) (July 2022)

**INTERNATIONAL SEARCH REPORT**

International application No.
**PCT/CN2023/135845**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/135845**

| Patent document cited in search report | Publication date (day/month/year) | Patent family member(s) | Publication date (day/month/year) |
|---|---|---|---|
| CN 113717246 A | 30 November 2021 | None | |
| WO 2022040607 A1 | 24 February 2022 | US 2023348378 A1 | 02 November 2023 |
| | | CA 3190091 A1 | 24 February 2022 |
| | | CN 116209433 A | 02 June 2023 |
| CN 107207564 A | 26 September 2017 | SG 11201703803 WA | 29 June 2017 |
| | | US 2018125988 A1 | 10 May 2018 |
| | | IL 251823 A0 | 31 July 2017 |
| | | PH 12017500866 A1 | 06 November 2017 |
| | | MX 2017006016 A | 19 June 2017 |
| | | EA 201790871 A1 | 30 November 2017 |
| | | AU 2015346330 A1 | 11 May 2017 |
| | | EP 3218390 A2 | 20 September 2017 |
| | | EP 3218390 A4 | 21 November 2018 |
| | | JP 2018500049 A | 11 January 2018 |
| | | WO 2016077505 A2 | 19 May 2016 |
| | | WO 2016077505 A3 | 21 July 2016 |
| | | CA 2964968 A1 | 19 May 2016 |
| | | KR 20170083095 A | 17 July 2017 |
| | | BR 112017009951 A2 | 26 December 2017 |
| CN 109416359 A | 01 March 2019 | JP 2019522625 A | 15 August 2019 |
| | | WO 2017192605 A1 | 09 November 2017 |
| | | US 2019134240 A1 | 09 May 2019 |
| | | US 11857648 B2 | 02 January 2024 |
| | | AU 2017260260 A1 | 22 November 2018 |
| | | AU 2017260260 B2 | 14 September 2023 |
| | | KR 20190003722 A | 09 January 2019 |
| | | US 2019134239 A1 | 09 May 2019 |
| | | WO 2017192598 A1 | 09 November 2017 |
| | | AU 2023282172 A1 | 04 January 2024 |

Form PCT/ISA/210 (patent family annex) (July 2022)